(19) Europäisches Patentamt
European Patent Office
Office européen des brevets

(11) EP 1 998 804 B1

(12) EUROPEAN PATENT SPECIFICATION

(45) Date of publication and mention
of the grant of the patent:
16.04.2014 Bulletin 2014/16

(51) Int Cl.:
*A61K 39/39* (2006.01)   *A61P 37/04* (2006.01)

(21) Application number: 07727347.2

(22) Date of filing: 26.03.2007

(86) International application number:
PCT/EP2007/052874

(87) International publication number:
WO 2007/110409 (04.10.2007 Gazette 2007/40)

(54) **COMPOSITIONS COMPRISING A RECOMBINANT ADENOVIRUS AND AN ADJUVANT**

ZUSAMMENSETZUNGEN, DIE EINEN REKOMBINANTEN ADENOVIRUS UND EIN ADJUVANS UMFASSEN

COMPOSITIONS COMPRENANT UN ADÉNOVIRUS RECOMBINÉ ET UN ADJUVANT

(84) Designated Contracting States:
AT BE BG CH CY CZ DE DK EE ES FI FR GB GR
HU IE IS IT LI LT LU LV MC MT NL PL PT RO SE
SI SK TR

(30) Priority: 27.03.2006 EP 06111760
27.03.2006 US 786147 P
29.06.2006 EP 06116318

(43) Date of publication of application:
10.12.2008 Bulletin 2008/50

(73) Proprietor: Crucell Holland B.V.
2333 CN Leiden (NL)

(72) Inventors:
• HAVENGA, Menzo Jans Emko
2333 CN Leiden (NL)
• RADOSEVIC, Katarina
2333 CN Leiden (NL)

(74) Representative: Verhage, Richard Abraham et al
Crucell Holland B.V.
IP Department
Archimedesweg 4-6
2333 CN Leiden (NL)

(56) References cited:
WO-A-2004/055187   WO-A-2006/040334

• OPHORST O J A E ET AL: "Immunogenicity and protection of a recombinant human adenovirus serotype 35-based malaria vaccine against Plasmodium yoelii in mice" INFECTION AND IMMUNITY, vol. 74, no. 1, January 2006 (2006-01), pages 313-320, XP002437227 ISSN: 0019-9567
• KURTIS J D ET AL: "Pre-erythrocytic immunity to Plasmodium falciparum: The case for an LSA-1 vaccine" TRENDS IN PARASITOLOGY, ELSEVIER SCIENCE, AMSTERDAM, NL, vol. 17, no. 5, May 2001 (2001-05), pages 219-223, XP002272410
• HERRINGTON D A ET AL: "SAFETY AND IMMUNOGENICITY IN VOLUNTEERS OF A RECOMBINANT PLASMODIUM-FALCIPARUM CIRCUMSPOROZOITE PROTEIN MALARIA VACCINE PRODUCED IN LEPIDOPTERAN CELLS" VACCINE, vol. 10, no. 12, 1992, pages 841-846, XP002437228 ISSN: 0264-410X
• BREWER ET AL: "(How) do aluminium adjuvants work?" IMMUNOLOGY LETTERS, AMSTERDAM, NL, vol. 102, no. 1, 15 January 2006 (2006-01-15), pages 10-15, XP005157442 ISSN: 0165-2478
• O'HAGAN D T ET AL: "RECENT ADVANCES IN VACCINE ADJUVANTS: THE DEVELOPMENT OF MF59 EMULSION AND POLYMERIC MICROPARTICLES" MOLECULAR MEDICINE TODAY, ELSEVIER, CAMBRIDGE, GB, vol. 96, February 1997 (1997-02), pages 69-75, XP002977233 ISSN: 1357-4310

- **HILGERS LUUK A T ET AL: "Sucrose fatty acid sulphate esters as novel vaccine adjuvant. AVAILABLE ONLINE ON 7.02.2005" VACCINE 12 APR 2006, vol. 24 Suppl 2, 7 February 2005 (2005-02-07), pages S2-81, XP002437229 ISSN: 0264-410X**

**Description**

FIELD OF THE INVENTION

The invention relates to the field of medicine. In particular, it relates to the field of vaccination using viral vectors. More in particular, the invention relates to a (pharmaceutical) composition comprising a recombinant, replication-defective adenoviral vector in combination with an adjuvant, and the use thereof.

BACKGROUND OF THE INVENTION

Therapeutic and prophylactic treatment through vaccination has been applied widely over the last century, covering a wide range of infectious diseases caused by numerous pathogenic organisms. Much attention was drawn over the last two decades to the use of (recombinant, and preferably non-replicating) viral vectors as delivery means for transferring a compound (generally in the form of a nucleic acid encoding an antigen) to a subject, and more specifically, and preferably, targeting towards antigen presenting cells within that subject. One type of viral vector that has been studied and for which several clinical trials are presently ongoing, are based on recombinant adenoviruses.

Vaccines comprising recombinant adenoviruses carrying a heterologous antigen are known and investigated in the art for their use in vaccination. Recombinant adenoviral vectors are also used in gene therapy, or in tumor vaccination. Examples of vaccine compositions against infectious diseases are vaccines compositions comprising viruses based on Ad5 carrying HIV antigens for the therapeutic and prophylactic treatment of AIDS (Shiver et al. 2002. Nature 415:331-335), Ad5-based vectors carrying antigens from Ebola Virus (Sullivan et al. 2000. Nature 408:605-609), Ad35-based vectors carrying antigens from for instance *Mycobacterium tuberculosis* (against TB; application PCT/EP2005/055984) or *Plasmodium falciparum* (against malaria; WO 2004/055187; Heppner et al. 2005. Vaccine 23:2243-2250*;* Ophorst et al. 2006. Infect. Immun. 74:313-320).

Clearly, vaccines based on the recombinant viral technology provide a very good alternative for numerous vaccines based on conventional techniques, such as the well known inactivated pathogens, sub-unit vaccines or synthetic compounds. As such, there is a constant need in the field for improvements of the viral-based vaccine technology and particularly of the very promising adenoviral-based technology to provide even better compositions with improved and stronger antigenicity and prolonged immune responses.

BRIEF DESCRIPTION OF THE DRAWINGS

Figure 1 shows (A) the cell viability and eGFP expression, and (B) the number of viral particles determined upon infection with eGFP expressing viruses in the presence of increasing amounts of aluminium hydroxide (ALOH, in the form of Alhydrogel).
Figure 2: idem to Figure 1, for aluminium phosphate (ALPO: Adju-phos).
Figure 3: idem to Figure 1, for Covaccine HT.
Figure 4: idem to Figure 1, for MF59.
Figure 5 shows the T-cell response towards the circumsporozoite (CS) antigen from *P. falciparum* encoded by a transgene incorporated in a recombinant Ad35 virus after injection in mice in the absence or presence of aluminium hydroxide using a dominant epitope in the CS protein (A) and using a peptide pool covering the CS protein (B).
Figure 6: idem to Figure 5, for aluminium phosphate.
Figure 7 shows the antibody response towards the CS antigen from P. *falciparum* encoded by a transgene incorporated in a recombinant Ad35 virus after injection in mice in the absence or presence of aluminium hydroxide, determined after 4 and 8 weeks upon injection.
Figure 8: idem to Figure 7, for aluminium phosphate.
Figure 9 shows the cellular immune response towards the adenoviral particle in mice injected with a recombinant Ad35 virus in the absence or presence of aluminium hydroxide, at 4 weeks and at 8 weeks upon injection.
Figure 10: idem to Figure 8, for aluminium phosphate.
Figure 11 shows the antibody response in mice towards the viral particle (the transgene carrier) in the presence or absence of aluminium hydroxide at week 4 and week 8 upon injection.
Figure 12: idem to Figure 11, for aluminium phosphate.
Figure 13: idem to Figure 5, for Covaccine HT.
Figure 14: idem to Figure 5, for MF59.
Figure 15: idem to Figure 7, for Covaccine HT.
Figure 16: idem to Figure 7, for MF59.

Figure 17: idem to Figure 9, for Covaccine HT.

Figure 18: idem to Figure 9, for MF59.

Figure 19: idem to Figure 11, for Covaccine HT.

Figure 20: idem to Figure 11, for MF59.

Figure 21 shows the T-cell response against the SIVGag antigen after injection of Ad35.SIVGag (A), Ad5.SIVGag (B) and Ad49.SIVGag (C) in three different doses $\pm$ aluminium phosphate.

Figure 22 shows the Neutralizing antibody (Nab) titers directed against the viral particle after injection of Ad35.SIVGag (A), Ad5.SIVGag (B) and Ad49.SIVGag (C) in three different doses $\pm$ aluminium phosphate.

Figure 23 shows the T-cell response against the SHIVEnv antigen after injection of Ad35.SHIVEnv (A), Ad5.SHIVEnv (B) and Ad49.SHIVEnv (C) in three different doses $\pm$ aluminium phosphate.

Figure 24 shows the antibody response against the SHIVEnv antigen after injection of Ad35.SHIVEnv (A), Ad5.SHIVEnv (B) and Ad49.SRIVEnv (C) in three different doses $\pm$ aluminium phosphate ($10^7$ vp dose for Ad49.SHIVEnv not shown).

Figure 25 shows the antibody response (A) and T-cell response (B) against the CS antigen after a single administration of Ad35.CS, or in a prime/boost administration, in the presence or absence of aluminium phosphate.

Figure 26 shows the adsorption study with Ad35.CS using aluminium hydroxide (A) or aluminium phosphate (B).

Figure 27 shows the LSA-1 specific T cell response after 4 weeks in mice that were injected with Ad35.CL with or without aluminium phosphate.

Figure 28 shows the LSA-1 specific antibody response after 2, 4, 6 and 8 weeks in mice that were injected with Ad35.CL with or without aluminium phosphate.

SUMMARY OF THE INVENTION

The present invention relates to the subject matter of the claims.

DETAILED DESCRIPTION

The inventors of the present invention have found that the potency of adenoviral-based vaccines is significantly improved by adding an adjuvant to the compositions that comprise the recombinant adenovirus carrying a nucleic acid encoding an antigen. It was found that the immune response towards the antigen was significantly increased upon the addition of the adjuvant. Importantly, both cellular and humoral immune responses were increased.

Adjuvants are known immune response potentiators and have been widely applied for many years to increase the immune response of antigenic compositions. Examples of adjuvants that have been used for many years and that are approved for human applications, are mainly those based on aluminium (also referred to as 'alum'): aluminium hydroxide and aluminium phosphate. Other well-known adjuvants that are applied in animals are Freund's complete (or incomplete) adjuvants. In recent years, many new adjuvant compounds have been found, or developed.

Without wishing to be bound by theory, in general the main mechanisms in which the adjuvants work are typically seen as 1) retaining the antigen at the site of injection, 2) causing a mild inflammation at the site of injection, 3) causing the recruitment of dendritic cells towards the site of injection, 4) inducing the uptake of antigen by the dendritic cells, and 5) promoting the maturation of dendritic cells, or combinations of two or more of the above.

Examples of adjuvants include compounds from the following categories:

Mineral Containing Compositions

Mineral salts such as aluminium salts and calcium salts, hydroxides (e.g. oxyhydroxides), phosphates (e.g. hydroxyphosphates, orthophosphates), and sulfates, or mixtures thereof (*see* Vaccine Design. 1995. eds. Powell & Newmann. Plenum). Generally used adjuvants from this category are aluminium hydroxide, alum and Alhydrogel, which is an aluminium hydroxide gel. Also aluminium phosphate is widely applied; Adju-phos is one example of an aluminium phosphate adjuvant.

It is to be understood that the general term 'aluminium hydroxide adjuvant' actually refers to a compound which should be noted (more correctly) as 'aluminium oxyhydroxide' [chemically: AlO(OH)], which is a compound in a crystalline form with surface OH groups, an iso-electric point of 11.4 and a surface charge at pH 7.4. On the other hand, it should also be understood that the general term 'aluminium phosphate adjuvant' as used generally herein, is also a misnomer as the more correct name should be 'aluminium hydroxyphosphate' [chemically: $Al(OH)_m(PO_4)_n$], which is a compound in a crystalline form with both OH and $PO_4$ surface groups. It has an iso-electric point of 4-6 and a surface charge at pH 7.4. Thus, also aluminium phosphate adjuvant has OH groups at its surface. Adju-phos is an example of an aluminium phosphate based adjuvant and the name of the aluminium phosphate adjuvants commonly used by persons skilled in the art. ALPO is an abbreviation for aluminium phosphate. ALOH is an abbreviation for aluminium hydroxide. Alhydrogel is the common name for a gel of aluminium hydroxide as it is generally applied in the art.

Without wishing to be bound by theory, the way these adjuvants are believed to work is that the antigen is generally trapped inside the aggregates holes in either compound. The aluminium hydroxide generally forms aggregates of approximately 17 µm, whereas the aluminium phosphate adjuvant generally forms aggregates.of approximately 3 µm. Both forms do hardly go into solution (appr. 1 ppm). By mixing the antigen with the adjuvant it may relatively easy be tested whether an antigen is considered adsorbed to the adjuvant, by subsequently spinning the aggregates (yes or no including the antigen) and measure the amount of antigen left in the supernatant. Depending on numerous factors, but also on the phosphorylation status of the antigen, the antigen becomes more or less adsorbed onto the adjuvant.

In view of the present invention, as the antigen itself (the proteinaceous antigen encoded by the nucleic acid present in the genome of the recombinant adenovirus) is not present together with the adjuvant, it is postulated that adsorption is less relevant or even not required at all for a proper immune response. The antigen becomes expressed after the virus has entered a host cell in the body of the host and whether the adjuvant was adsorbed onto the viral particle is not crucial for its direct action on the antigen. It may even be that when the antigen is less adsorbed, the adjuvant activity may be higher, since adsorption to the viral particle itself may hamper the final immunogenic stimulatory effect on the subsequently produced antigen.

Oil-Emulsions

Oil-emulsion compositions (or oil-in-water compositions, as also used herein) suitable for use as adjuvants include squalene-water emulsions, such as MF59 (5% Squalene, 0.5% Tween-80, 0.5% Span 85, formulated into submicron particles using a microfluidizer, see WO 90/14837; Podda A. 2001. Vaccine 19:2673-2 680) or submicron oil-in-water emulsions based on MF59. Other submicron oil-in-water emulsions are MF75 (or SAF) and Covaccine HT. Complete Freund's adjuvant and incomplete Freund's adjuvant are oil-emulsion-based adjuvant compositions, and may also be used.

Herein, the term 'adjuvant' relates to a compound, which is present together with the recombinant adenovirus in a composition, wherein the adjuvant stimulates the immune response towards the antigenic substance encoded by the transgene located in the genome carried by the recombinant adenovirus.

The adjuvant generally does not comprise the antigenic determinant itself. The adjuvant stimulates the immune response against the antigenic determinant, which response would be lower if the adjuvant would not have been present. It is preferred that the adjuvant is non-toxic to the host and the cells within the host at least at normal dosages and in accordance with the contemplated administration regime. It is also preferred that the adjuvant stimulates at least the cellular- and/or the humoral immune response against the antigenic determinant.

However, the present invention relates to the combination of a recombinant adenoviral vector and an (preferably purified) adjuvant present in one composition, wherein the adjuvant is preferably chosen from the group consisting of aluminium phosphate, CoVaccine HT and MF59. It also relates to kits comprising the adenovirus and the adjuvant in separate vials to be brought together in a single composition that can be administered to a host. Several applicators are used in the art, such as dual delivery needles that have two separated compartments, which ensure that only at the exact time of delivery in the host, such compositions are brought together, hence at the exact moment of administration.

The adjuvant itself is mixed with the recombinant adenovirus in the composition. The adjuvant may be bound to the adenovirus, for instance through adsorption. When aluminium phosphate is used, no adsorption, or no significant adsorption, is preferred.

Binding may be provided through covalent bonds. When the recombinant adenovirus is covalently bound to the adjuvant, it is generally made beforehand by covalently linking the adenovirus to the adjuvant with a suitable activator. Methods for binding two components with a covalent bond are well known in the art.

The adjuvant and recombinant vector may also be administered separately, either simultaneously or subsequently, as long as the site of administration of the adjuvant and the recombinant vector (partially) overlap. The adenovirus particle itself may also contribute to the immune response directed against the encoded antigenic determinant.

The use of adjuvants in a composition with recombinant viruses has been suggested in the art. WO 03/037275 relates to the combination of recombinant adenoviruses with adjuvants, and discloses a long list of possible adjuvants: Freund's incomplete- and complete adjuvants, Merck Adjuvant 65, AS-2, a salt of calcium, iron or zinc, an insoluble suspension of acylated tyrosine acylated sugars, cationically or anionically derivatized polysaccharides, polyphosphazenes, biodegradable microspheres, aminoalkyl glucosaminide phosphates, monophosphoryl lipid A, saponins, water/oil adjuvants and mixtures thereof (see claims 2-6 of WO 03/037275). Also, the use of aluminium hydroxide and aluminium phosphate was mentioned in passing. However, this publication lacks any specific disclosure of recombinant adenoviruses in one composition with an adjuvant. It suggests its use but does not show which of the adjuvants is preferred or actually stimulates an immune response. The inventors of the present invention herein now disclose that the action of an adjuvant in the context of an adenovirus serotype occurs and is moreover serotype specific. Notably, the inventors show here that not all adjuvants (such as those in the long list mentioned in WO 03/037275) do stimulate the immune response when present in a composition with a recombinant adenovirus. Actually, it has surprisingly been found and now disclosed here for the first time that aluminium phosphate based adjuvants do stimulate such a response, while aluminium hydroxide based adjuvants do not, at least when applied in the context of a recombinant adenovirus serotype 35. The art is silent on the herein disclosed difference between aluminium hydroxide and aluminium phosphate, and is silent on serotype specificity with respect to adenoviruses in the context of adjuvants.

It is now shown here that aluminium phosphate stimulates the immune response, while aluminium hydroxide does not. Aluminium phosphate functions as an adjuvant when used together with Ad35-based vectors, while Ad5- and Ad49-based vectors do not benefit from the addition of this compound. Such serotype-specific effects are very surprising.

The present invention relates to a composition comprising a recombinant replication-defective adenovirus serotype 35 and an adjuvant. Such compositions are applicable for different uses, preferably for vaccination. 'Replication-defective' (or replication-incompetent, or replication-deficient) means that the adenovirus cannot replicate in normal (or non-packaging) cells. Generally, they can only be produced, replicated and packaged in so-called packaging cells, that provide all necessary components required for replication, which components are missing from the replication-defective adenovirus. The composition of the present invention is preferably a pharmaceutical composition, and preferably further comprises a suitable excipient and/or diluent. The term 'adjuvant' should be interpreted broadly. It refers to compounds that stimulate (or increase) immune responses. Many compounds in the art are known that stimulate immune responses, and have been used for decades. Well-known examples are Freund's (in)complete adjuvant, tetanus toxoid and aluminium based adjuvants. Even inactive flaviviruses can act as adjuvants under specific circumstances (see EP 0833923 B1). In general, an adjuvant is a compound, a natural or synthetic substance that increases the immune response towards an antigen when the antigen and the adjuvant are administered together, or at the same time or are both present in the host. Adjuvants are widely applied to stimulate and increase immune responses. Although the way through which adjuvants actually work remains in certain cases obscure, several adjuvants are known to stimulate the activity and development of T cells and the production of neutralizing antibodies.

In a preferred embodiment, the invention relates to a composition, wherein said adjuvant is an aluminium phosphate-based adjuvant. The adjuvant is present in the composition according to the present invention in a concentration generally applied in the art. Preferably a concentration in the range of 0.1 to 10 mg/ml is used, and more preferably a concentration in the range between 0.1 to 2.5 mg/ml is used.

In another preferred embodiment, the invention relates to a composition according to the invention, wherein said adjuvant is an oil-emulsion adjuvant. As outlined above, several oil-emulsion-based adjuvants (or water-in-oil adjuvants) are known. Preferably, said oil-emulsion adjuvant is Covaccine HT and/or MF59. Although such adjuvants are generally used on their own in combination with the antigen or in the case of the present invention in the presence of the carrier (the gene delivery vehicle, or the recombinant adenovirus), they may also be applied in combinations, where one of the adjuvants stimulates the cellular immune response and wherein the other adjuvant may have a more profound effect on the humoral immune response.

In one particular embodiment of the invention, the invention relates to a composition according to the invention, wherein said adenovirus comprises an adenoviral genome comprising a heterologous nucleic acid of interest. A 'heterologous nucleic acid' refers to a nucleic acid (generally DNA) that is non-adenoviral. Particularly preferred heterologous nucleic acids are gene of interest encoding antigenic determinants towards which an immune response needs to be raised. Such antigenic determinants are also typically referred to as antigens. Generally speaking, antigens are peptides, polypeptides or proteins from organisms that generally cause a disease. Therefore, in a further preferred embodiment, said heterologous nucleic acid of interest encodes an immunogenic determinant. More preferably, said immunogenic determinant is an antigen from a bacterium, a virus, yeast or a parasite. The diseases caused by such organisms are generally referred to as 'infectious disease' (and are thus not limited to organisms that 'infect' but also to those that enter the host and cause a disease. So-called 'self-antigens', e.g. tumour antigens, also form part of the state of the art. Preferred examples from which the antigenic determinants (or antigens) are taken are malaria-causing organisms, such as *Plasmodium falciparum*, tuberculosis-causing organism such as *Mycobacterium tuberculosis*, yeasts, or viruses. Particularly, antigens from viruses such as flaviviruses (e.g., West Nile Virus, Hepatitis C Virus, Japanese Encephalitis Virus, Dengue Virus), ebola virus, Human Immunodeficiency Virus (HIV), and Marburg virus may be used in compositions according to the present invention. In one particularly preferred embodiment, said antigen is the CS protein from *P. falciparum*. In another embodiment, the antigenic determinant is a protein of one antigen-, or a fusion protein of several antigens from *M. tuberculosis*, such as the Ag85A, Ag85B and/or the TB10.4 antigens (see for the construction and production of such TB vaccine viruses WO 2006/053871).

In yet another preferred embodiment, said immunogenic determinant is a viral glycoprotein, such as GP from ebola virus or Marburg virus.

In a preferred embodiment, the invention relates to a composition in which the recombinant adenovirus is mixed with an aluminium phosphate adjuvant and wherein the antigen is the circumsporozoite (CS) protein, or a immunogenic part thereof, of a malaria causing pathogen, preferably *P. falciparum.* In another preferred embodiment, said CS antigen, or said immunogenic part thereof, is present in said recombinant adenovirus with another malaria-related antigen, such as the Liver Specific Antigen-1 (LSA-1) protein. These antigens may be linked to form a single transcript from a single expression cassette or may be present in two separate expression cassettes cloned in different parts of the adenoviral genome. Most preferred is a composition comprising a recombinant adenovirus wherein the E1 region has been deleted and replaced by an expression cassette comprising a heterologous promoter providing the expression of a transgene encoding the CS antigen alone or linked to a transgene encoding the LSA-1 antigen.

The compositions of the present invention are preferably used in the treatment (be it prophylactically or post-infection, i.e. therapeutically) of animals and humans. Therefore, in one preferred aspect, the compositions of the present invention further comprise a pharmaceutically acceptable excipient. Such pharmaceutically acceptable excipients (or carriers) are known in the art and are widely applied. Generally, buffered solutions are applied.

Since adenoviruses cause common cold in humans, and many adenovirus serotypes have infected most of the human world population at a certain age, it is preferred to use a human adenovirus serotype that encounters low levels of neutralizing antibodies in the host. Such low-neutralized serotypes are mainly found in the adenoviruses of subgroup B and D. The serotype according to the present invention and that encounters neutralizing activity in only a small percentage of sera from individuals around the world, is the subgroup B adenovirus Ad35.

For safety reasons, as well as providing space in the viral genome to incorporate genes of interest, the adenovirus is made replication-defective by removal of all or at least most of the functional parts of the E1 region of the adenoviral genome. The E3 region is preferably removed, as it does not play an essential role for replication or packaging, and can therefore be removed to allow larger inserts to be incorporated into the adenoviral genome. To enable the production of subgroup B and D viruses on Ad5-E1 transformed cell lines such as the PER.C6® cells and 293 cells, it is preferred that the E4 or f6 region in the subgroup B or D virus is from a subgroup C adenovirus, preferably Ad5. This makes that the E4 or f6 region is compatible with the E1.55K protein encoded by the E1 region present in the packaging cell, and ensures high titers during production. This technology is outlined in WO 03/104467.

The invention also relates to the use of aluminium phosphate in the preparation of a medicament comprising a recombinant replication-defective adenovirus, for the diagnosis, prophylaxis or treatment of an infectious disease. As mentioned above, the term 'infectious disease' relates to diseases caused by external organisms, such as bacteria, yeasts, viruses or parasites. In a preferred setting, the compositions of the present invention are useful in the treatment of infectious disease caused by P. *falciparum* (malaria).

In yet another aspect, the invention relates to the use of aluminium phosphate in prime/boost regimens, in which both the priming composition as well as the boosting composition contains aluminium phosphate. In a homologous prime/boost setup the priming and boosting composition are the same and contain the same adenovirus serotype, whereas in a heterologous prime/boost setup the priming composition contains a recombinant viral vector of a different type or serotype than the recombinant viral vector in the boosting composition. The viral vectors may be picked from recombinant adenoviruses and its respective serotypes, as well as from recombinant alphaviruses, vaccinia viruses and influenza viruses. Preferably, when an adenovirus is selected, so-called low-neutralized viruses (or 'rare' viruses, such as Ad11, Ad26, Ad35, Ad48 and Ad49) are selected for priming and/or boosting.

A further advantage of stronger immune responses against the antigenic determinant in adjuvanted (adeno) viral vectored vaccines is in the possibility to reduce the viral titer needed for vaccination. This reduces costs of goods for these viral vector vaccines, but it also reduces pain or unwanted side effects in recipients.

The invention also relates to the use of an oil-emulsion adjuvant in the preparation of a medicament comprising a recombinant replication-defective adenovirus serotype 35, for the diagnosis, prophylaxis or treatment of an infectious disease. Said oil-emulsion adjuvant is Covaccine HT or MF59.

The invention also discloses the use of an adjuvant in the preparation of a medicament, said medicament further comprising a recombinant replication-defective adenovirus serotype 35, wherein said adenovirus comprises a heterologous nucleic acid of interest, for increasing the immune response towards an antigen encoded by said heterologous nucleic acid. For the sake of clarity: the adjuvant is used to make the medicament and for increasing (stimulating) the immune response towards an antigen encoded by said heterologous nucleic acid, present in said adenovirus. Preferably, said adjuvant is an oil-emulsion adjuvant. More preferably, said oil-emulsion adjuvant is Covaccine HT or MF59. In another preferred embodiment, said adjuvant is an aluminium phosphate based adjuvant.

The invention also discloses the use of a composition according to the invention in the preparation of a medicament, for the diagnosis, prophylaxis or treatment of an infectious disease.

The invention furthermore relates to a kit of parts comprising a recombinant replication-defective adenovirus serotype 35, said adenovirus comprising a genome comprising a heterologous nucleic acid of interest; and an adjuvant. Preferably, said adjuvant is an aluminium-based adjuvant or an oil-emulsion adjuvant. More preferably, said adjuvant is selected from the group consisting of aluminium phosphate, Covaccine HT and MF59.

The adjuvants and recombinant adenoviruses may also be used in heterologous prime-boost set ups, wherein the first applied adenovirus is followed by another adenovirus of a different serotype than the initially applied virus. Either of these subsequently administered recombinant adenoviruses may be combined with the same or with different adjuvants, resulting also in a homologous or heterologous prime-boost set up with respect to the applied adjuvants: adenovirus-1 + Adjuvant-X followed by adenovirus-2 + Adjuvant-Y, wherein 1 is a different adenovirus from 2 and wherein X may be the same or different from Y.

It is concluded here that, although the immuno-potentiating effects of aluminium-based adjuvants were known in the art for non-viral vector vaccines, and although it has been mentioned to use different kinds of adjuvants (including those based on aluminium) in a composition with recombinant adenoviruses, it was not disclosed, nor made credible, that an effect on antigen response would be observed, let alone that an aluminium phosphate based adjuvant shows extraordinary results in a composition with a recombinant adenoviral vector of a specific serotype. The fact that Ad35 turned out to be the serotype of choice as it could be stimulated in its immunostimulating effects by using aluminium phosphate, while other serotypes did not benefit that much, or at all, was very surprising. Even though it was suggested in WO 03/037275 to combine a vaccine based on an adenoviral vector with an adjuvant, the studies provided and disclosed herein now show that only a small subset of all mentioned adjuvants are really applicable, and even in a serotype-dependant manner. Importantly, aluminium hydroxide adjuvant does not appear to have a positive effect on the immune response, whereas an aluminium phosphate-based adjuvant does. This surprising difference in effect between these two aluminium-based adjuvants was not anticipated in the art.

As disclosed herein, the positive effect of aluminium phosphate is apparently linked to certain adenovirus serotypes. It is disclosed that the immune response (antibodies as well as T cells) towards the CS and the SIVGag antigens is positively enhanced in combination with Ad35, and that the immune response (antibodies) towards the SHIVEnv antigen also benefits from the addition of aluminium phosphate. Such effects cannot be determined when the antigens are presented by two other serotypes that were studied: Ad5 and Ad49.

These differences, although not fully understood, may be explained by the effects towards the viral vector itself, hampered in its infectivity capacity (by the addition of the adjuvant) or hampered in its ability to express and sustain the traffic and routing of the antigen in the host cell. The invention relates to a vaccine composition that is suitable for the prophylactic and/or therapeutic treatment of malaria, said vaccine composition comprising a recombinant, replication-defective adenoviral vector based on serotype 35 (Ad35), and further comprising an aluminium phosphate adjuvant, wherein the adenoviral vector preferably comprises a nucleic acid encoding the CS antigen, or an immunogenic part thereof, incorporated into its genome.

The invention relates to a composition comprising a recombinant replication-defective serotype 35 adenovirus (Ad35) comprising an adenoviral genome, said genome comprising a heterologous nucleic acid encoding an immunogenic determinant; and an aluminium-phosphate based adjuvant. Preferably, said immunogenic determinant is the circumsporozoite (CS) protein, or an immunogenic part thereof, from a malaria-causing pathogen. In another preferred embodiment, said immunogenic determinant is the LSA-1 protein, or an immunogenic part thereof, from a malaria-causing pathogen. In another preferred embodiment said CS protein or said immunogenic part thereof is combined with the LSA-1 protein, or an immunogenic part thereof, from a malaria-causing pathogen. Preferably, said malaria-causing organism is *Plasmodium falciparum.*

In one aspect of the invention the nucleic acids encoding said CS protein and said LSA-1 protein, or their respective immunogenic parts, are linked to encode a single transcript, said single transcript providing a fusion protein.

In a preferred embodiment, the invention relates to a composition according to the invention, wherein said replication-defective adenovirus comprises an adenoviral genome comprising a deletion in the E1 region rendering the adenovirus replication-defective, and further preferably comprising a deletion of the E3 region. In a preferred embodiment, said genome further comprises an E4 or f6 region from adenovirus serotype 5. For therapeutic applications, the composition prefereably further comprises a pharmaceutically acceptable excipient and/or diluent, which are compounds generally applied in the art.

The invention also relates to a use of a composition according to the invention in the preparation of a medicament for the diagnosis, prophylaxis or treatment of malaria.

The invention also relates to the use of an aluminium phosphate adjuvant in the preparation of a medicament, said medicament comprising a recombinant replication-defective adenovirus, for the diagnosis, prophylaxis or treatment of malaria. Said recombinant replication-defective adenovirus is based on Ad35.

The invention also relates to the use of an aluminium phosphate adjuvant in the preparation of a medicament, said medicament comprising a recombinant replication-defective Ad35, for the diagnosis, prophylaxis or treatment of an infectious disease. Preferably, said infectious disease is malaria. In a preferred aspect, said Ad35 comprises an adenoviral genome comprising a heterologous nucleic acid encoding a CS antigen, or an immunogenic part thereof, of *P. falciparum*, wherein it is further preferred that said adenoviral genome comprises a heterologous nucleic acid encoding an LSA-1 antigen, or an immunogenic part thereof, of *P. falciparum.*

The present invention also relates to the use of an oil-emulsion adjuvant in the preparation of a medicament, said medicament further comprising a recombinant replication-defective adenovirus, for the diagnosis, prophylaxis or treatment of malaria, wherein said oil-emulsion adjuvant is Covaccine HT or MF59. Said recombinant replication-defective adenovirus is based on human adenovirus serotype 35 (Ad35). More preferably, said adenovirus comprises an adenoviral genome comprising a heterologous nucleic acid of interest encoding the CS antigen, or an immunogenic part thereof, from *P. falciparum.* The invention also relates to a kit of parts comprising a recombinant replication-defective serotype 35 adenovirus (Ad35) comprising an adenoviral genome, said genome comprising a heterologous nucleic acid encoding an immunogenic determinant; and an aluminium-phosphate based adjuvant. Preferably, said immunogenic determinant is the CS protein, or an immunogenic part thereof, of *P. falciparum.* More preferably, said adenoviral genome further comprises a nucleic acid encoding an LSA-1 protein, or an immunogenic part thereof, of P. *falciparum.*

EXAMPLES

**Example 1. Preparation of a vaccine based on a replication incompetent recombinant Ad35**

An Ad35 prototype stock derived from the ATCC was propagated on PER.C6® cells (as represented by the cells as deposited at the European Collection of Cell Cultures, Porton Down, Salisbury, Wiltshire, SP4 0JG, UK, under ECACC number 96022940), and viral DNA was isolated using general techniques known to the person skilled in the art. The viral genome was sequenced using the shotgun method (see WO 00/70071) and is publicly available under Genbank Acc. No. AY271307. Recombinant adenoviruses lacking the E1 region (making the recombinant adenovirus replication-deficient), lacking the E3 region (providing cloning space) and comprising the codon-optimized circumsporozoite (CS) from *Plasmodium falciparum* have been described in detail elsewhere (WO 97/00326; WO 99/55132; WO 99/64582; WO 00/70071; WO 00/03029 and WO 04/055187). To enable production of the recombinant viruses on packaging cells, a single homologous recombination system was used, in which the adapter plasmid (including the heterologous gene and part of the left genomic region of the adenovirus) contains overlap with a cosmid vector, which comprises the remainder of the (right) adenoviral genome. The viruses were produced with the use of PER.C6 cells that have been modified to express the E1B-55K protein of Ad35 (see WO 00/70071 and WO 02/40665). If packaging cells are used that do not express the E1B-55K protein of a subgroup B adenovirus to produce subgroup B based adenoviruses, such as Ad35, it is preferred that the viral genome of the adenovirus is manipulated such that it contains the E4 or f6 region that is compatible with the E1B-55K protein expressed by the cell line. Preferably, the E4 or f6 region of Ad5 is used for this purpose (see WO 03/104467). Control viruses contained the green fluorescent protein-encoding gene, cloned into the E1 region of the adenoviral genome.

**Example 2. In vitro effect of adjuvant on rAd35-CS vaccine potency and toxicity**

Human lung carcinoma cells A549 (ATCC, Cat. no. CCL-185) were maintained in DMEM (Gibco BRL), 10% FCS (Gibco BRL) and penicillin/streptomycin (Gibco BRL) at 37°C and 10% $CO_2$. Cells were typically cultured in T175 tissue culture flasks and split 1:10 when cells reached approximately 70-90% confluence. For cell propagation A549 cells were washed once with 10 ml PBS per flask, subsequently trypsinized with 2 ml (1x) Trypsin and then re-suspended in fresh culture medium. First, the effect of adjuvant mixing with recombinant Ad35-CS with respect to vector viability and cell toxicity was studied. A cell suspension of $1 \times 10^5$ cells/ml was prepared and seeded in 24-well plates (1 ml/well).
Cells were incubated overnight. Infections were performed with typically a multiplicity of infection (m.o.i.) of $1 \times 10^3$ vp/cell. For this assessment, recombinant Ad35 viruses were used harboring the GFP encoding gene. Before infection, different adjuvant preparations were mixed with the recombinant vector. Four different adjuvant preparations, belonging to two different classes were tested.
The first set of adjuvants was aluminium-based; one being referred to as 'Alhydrogel (= aluminium hydroxide) and the other being generally referred to in the art as 'Adju-phos', which is an aluminium phosphate adjuvant. Both Alhydrogel (2.0%) as well as Adju-phos (2.0%) were purchased from Brenntag (Biosector Denmark) and formulations were used as specified in the certificate of analysis provided by the manufacturer. Alhydrogel (cat no. 28183000) was delivered in a concentration of 2%, with an aluminium concentration between 9 and 11 mg/ml, dissolved in a solution with a pH ranging between 6 and 7. Adju-phos (cat no. 283525100) was also delivered in a 2% concentration, containing aluminium in a concentration between 0.45 and 0.50% (= 3.5 - 5.0 mg/ml) in a solution with a pH between 6 and 7.
The second set of adjuvants was the so-called oil/water emulsion class of adjuvants. The two examples of such adjuvants that were tested herein are known in the art and are referred to as 'MF59' and 'CoVaccine™ HT'. Covaccine HT is marketed by CoVaccine B.V. (Utrecht, the Netherlands), whereas MF59 was developed by Chiron Corp.
Covaccine HT was obtained from CoVaccine B.V. and was made as described (Blom A.G. and Hilgers L.A.Th. 2004. Vaccine 23:743-754*).* The components were mixed in phosphate buffered saline pH 7.0, to contain 40 mg sucrose fatty acid sulphate ester, 40 mg Polysorbate 80 (ICI, London, UK) and 160 mg Squalene (Merck) per ml.
The MF59 adjuvant was also obtained from CoVaccine B.V. and was prepared as described (Higgins D.A. et al. 1996. Vaccine 14:478-484). The MF59 solution, in phosphate buffered saline pH 7.0, contains per ml: 5 mg (0.5% v/v) Span 85 (Sigma-Aldrich; cat. no. S7135), 5 mg (0.5% v/v) Polysorbate 80 (Sigma-Aldrich; cat. No. P8074) and 50 mg (5% v/v) Squalene (Sigma-Aldrich; cat. no. S3626).
Purchased stock solutions of Alhydrogel and Adju-phos were diluted in TRIS buffer (20 mM TRIS, 25 mM NaCl,

2 mM MgCl$_2$, 0.02% Tween-80, 10% sucrose, pH 8.0) to concentrations of 10, 6, 2, 1, 0.6 , 0.2 and 0.1 mg/ml or 5, 3, 2, 1, 0.6, 0.2 and 0.1, respectively. Dilutions were divided in two tubes and mixed with equal volumes (1:1) of either the TRIS buffer acting as negative control, or with rAd35.eGFP dissolved in the TRIS buffer. The recombinant Ad35-GFP vector and the respective adjuvant were mixed by rotation at 4°C for 30 min.

After mixing, 400 μl of each composition was added to A549 cells plated in 24-well plates (m.o.i. of 1x10$^3$ vp/cell for the viral infections) whereby each data point was measured 3 times in triplicate (9 wells) to determine:

- cell viability with Propidium Iodide (using a FACSCalibur from BD biosciences);
- infectivity by eGFP expression (using the FACSCalibur); and
- viral particles through Real Time PCR (Perkin-Elmer).

Hence, final concentration Alhydrogel and Adju-phos-on the cells was 5, 3, 1, 0.5, 0.3, 0.1 and 0.05 mg/ml for Alhydrogel and 2.5, 1.5, 1, 0.5, 0.3, 0.1 and 0.05 mg/ml for Adju-phos.

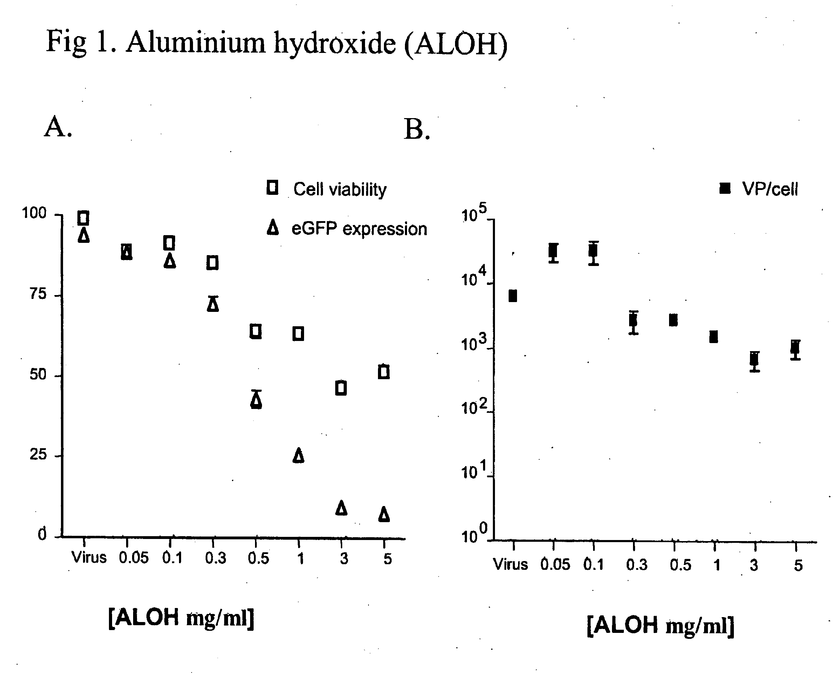

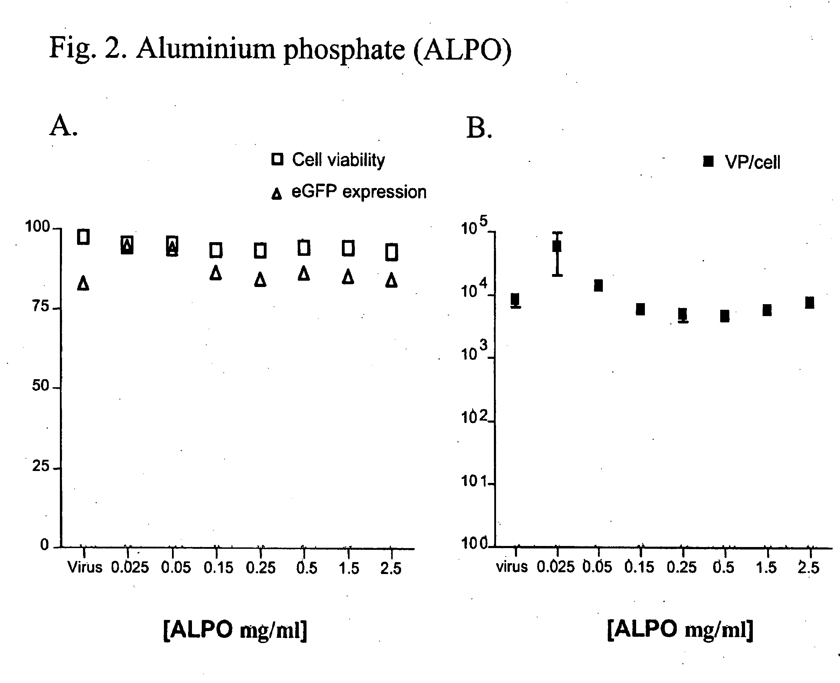

Exposure of cells to virus in presence or absence of aluminium hydroxide and aluminium phosphate was allowed to proceed for 2 h at 37°C after which virus/adjuvant solutions were removed by washing the cells once with PBS and DMEM Culture medium. Cells were then fed with fresh culture medium and cultured for another 24 h. Then, cells were harvested and monitored. For propidiumiodine (PI)-stainings and eGFP measurements cells were collected through trypsin treatment and transferred to tubes. Cells were centrifuged 5 min at 515 rcf, washed with PBA (PBS supplemented with 0.5% BSA) and resuspended in 200 μl 1:200 diluted PI solution (stock 1 mg/ml, Calbiochem) for testing cell viability. For GFP expression, cells were respuspended in 200 μl PBA and used in a FACS. To determine the infectivity via real time PCR, cells were harvested and transferred to tubes, centrifuged, resuspended in 200 μl PBS and stored at -80°C. The results in percentages on cell viability and eGFP as determined by FACs analysis (FACSCalibur) are provided in Figure 1A for Aluminium hydroxide and in Figure 2A for Aluminium phosphate. The calculations were made by comparing to the virus-only infection (left).

These data demonstrate that, when applying increasing concentrations of Aluminium hydroxide, eGFP expression declines, which is accompanied by a decrease in cell viability. This effect is already significantly present when applying a concentration of 0.5 mg/ml: this concentration of Aluminium hydroxide is typically used in clinical settings. Therefore, this concentration was chosen for *in vivo* testing (see below) given that only a two-fold decrease was observed in eGFP expression. Apparently, aluminium hydroxide in combination with recombinant adenoviral vectors have a negative effect on the expression of the transgene and exhibits toxic effects on the infected cells.

In contrast to the observation with aluminium hydroxide, no negative effect on cell viability or eGFP expression was observed when mixing aluminium phosphate with recombinant Ad35.eGFP (Figure 2A). Even at the highest concentration of 2.5 mg/ml no decrease in viability of protein expression was observed indicating that aluminium phosphate would provide a possible adjuvant candidate to use together with adenoviruses in adenoviral-based vaccines. In the *in vivo* experiments described below, also a concentration of 0.5 mg/ml was chosen for aluminium phosphate.

Besides cell viability and transgene-expression it was also addressed how many viral particles per cell could be determined after infection in the presence of adjuvants. For this, DNA was isolated from cells and Real-time PCR assays were performed essentially as described (verhaagh S. et al. 2006. J Gen Virol 87:255-265). Briefly, DNA was isolated from cells using the DNeasy tissue kit (Qiagen) and analyzed for the presence of Ad35 genomes using forward primer 5'-GTT CAG GGC CAG GTA GAC TTT G-3' (SEQ ID NO:1) and reverse primer 5'-CGC GGA AAT TCA GGT AAA AAA C-3' (SEQ IP NO:2), both primers recognizing sequences present within the CMV promoter present in rAd35. To determine the number of copies per cell, Q-PCR analyses were performed on the same samples using 18S rDNA sequences as targets for primers as described (Klein et al. 2000 Gene Ther 7:458-463). Results of the real time PCR analyses are depicted for aluminium hydroxide in Figure 1B and for aluminium phosphate in Figure 2B. These results coincide with the cell viability and eGFP expression data discussed above, showing that aluminium hydroxide negatively influences recombinant adenovirus infection and transgene expression, when the adjuvant is applied above concentrations of 0.1 mg/ml. In contrast, no effect on transgene expression, infection or cell viability upon infection with recombinant Ad35.eGFP was observed when using aluminium phosphate. Significantly, no such negative effects were observed even when the concentration of adjuvant was as high as 2.5 mg/ml.

Next to the experiments using the first class of adjuvants (the aluminium-based preparations), the second class based on oil-in-water emulsions were tested in a similar manner.

Again, A549 cells were seeded at 1x10$^5$ cells/ml per well in 24-well plates and incubated overnight. To determine an optimal dose of adjuvant, which would for instance be a loss of cell viability or virus infectivity less than 50%, stock solutions of MF59 and Covaccine HT were each diluted with TRIS buffer (20 mM TRIS, 25 mM NaCL, 2 mM MgCl$_2$, 0.02% Tween-80, 10% sucrose, pH 8.0) at a ratio of Adjuvant:Tris buffer = 10:1, 8:1, 4:1, 2:1, 1:1, 0.5:1, 0.25:1, 0.125:1. Dilutions were divided in two tubes and mixed with equal volumes (1:1) of TRIS buffer or recombinant Ad35.eGFP in TRIS buffer and mixed for 30 min at 4°C by rotation.

After incubation, 400 μl of each mixture was added to the plated cells (from which medium was removed) and subsequently cell viability, eGFP expression and viral particles were determined as described above for the aluminium-based adjuvant experiments. Final concentrations MF59 and Covaccine HT on the cell cultures were 5:1, 4:1, 2:1, 1:1, 0.5:1, 0.25:1, 0.125:1 and 0.0625:1.

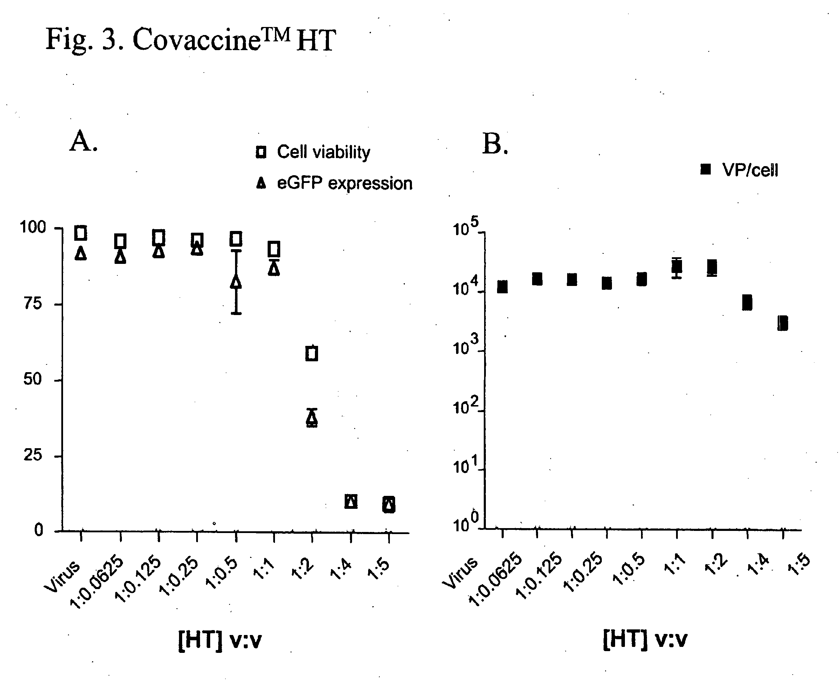

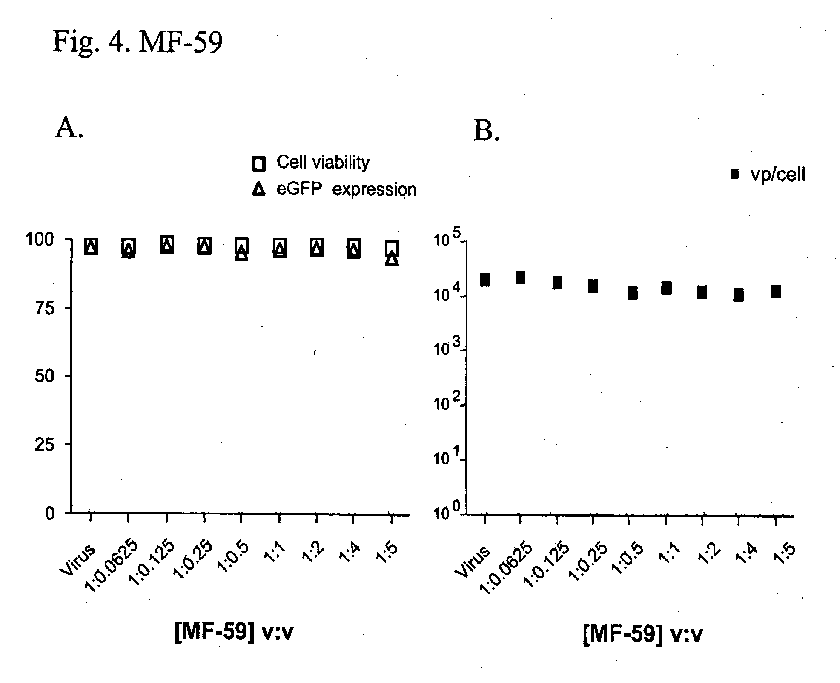

Infection was allowed to proceed for 2 h at 37°C, 10% $CO_2$ after which virus/adjuvant was removed by washing the cells once with PBS and DMEM Culture medium. Subsequently fresh culture medium was added. At day three, cells were harvested. Experiments to determine cell viability with PI, eGFP expression and cell infectivity via Real time PCR were performed as described above. Results of these experiments are shown in Figure 3 for Covaccine HT and in Figure 4 for MF59.

Notably, as with the aluminium-based adjuvants, also by using two examples from the water/oil emulsion class of adjuvants, dramatic differences were observed between the two examples. While Covaccine HT clearly has a negative impact on the cell viability and the GFP expression (Figure 3A), MF59 addition does not result in any negative effects with respect to these two parameters (Figure 4A). Moreover, Covaccine HT also has a negative impact on the number of adenoviral genomes per cell as shown in Figure 3B, when the concentration is above 1 v/v, although not as profound as Aluminium phosphate. Importantly, MF59 does not cause any negative effects on cell viability, transgene expression or copy number (Figure 4A and 4B), which indicates that important differences may exist between different adjuvants within the same class. Based on these results a ratio of 1 v/v is chosen for the *in vivo* experiments described below, as these concentrations are generally applied in literature (Ott et al. 1995 Vaccine 13:1557-1562).

## Example 3. Effect of an aluminium-based adjuvant on anti-insert specific T-cell responses *in vivo*

In order to test the effect of aluminium-based adjuvant preparations *in vivo,* mice (n=5 per time point; BALB/C, 6 weeks old, purchased from Harlan) were immunized with a rAd35-CS virus carrying a cDNA encoding for the circumsporozoite antigen of *Plasmodium falciparum* (rAd35-CS; see WO 2004/055187). rAd35-CS was diluted in TRIS buffer pH 8.0 using different vaccine doses (range of $2x10^7$, $2x10^8$, $2x10^9$ and $2x10^{10}$ vp/100 $\mu$l TRIS buffer. Dilutions were divided in two tubes and mixed with equal volumes (1:1) of TRIS buffer or Alum based adjuvant (Aluminium hydroxide or Aluminium phosphate diluted in TRIS buffer 0.5 mg/ml). The final concentration of adjuvant was thus 0.025 mg/ml in a total volume (including $10^7$, $10^8$, $10^9$ and $10^{10}$ vp rAd35-CS) of 100 $\mu$l (maximal volume suitable for intramuscular injection in mice). As control rAd35.empty was used (a recombinant replication-defective Ad35 virus carrying no antigen) with an end concentration of $10^{10}$ vp.

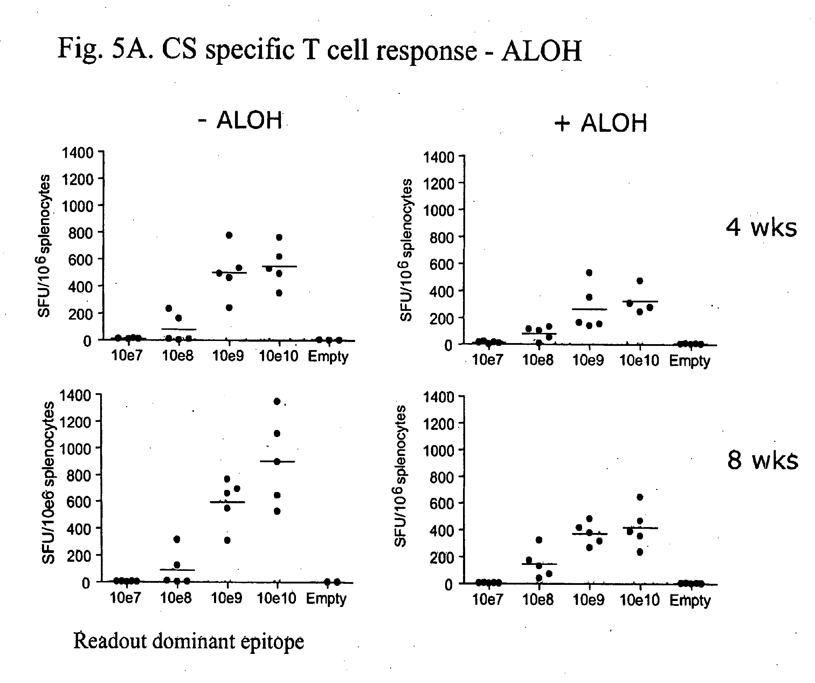

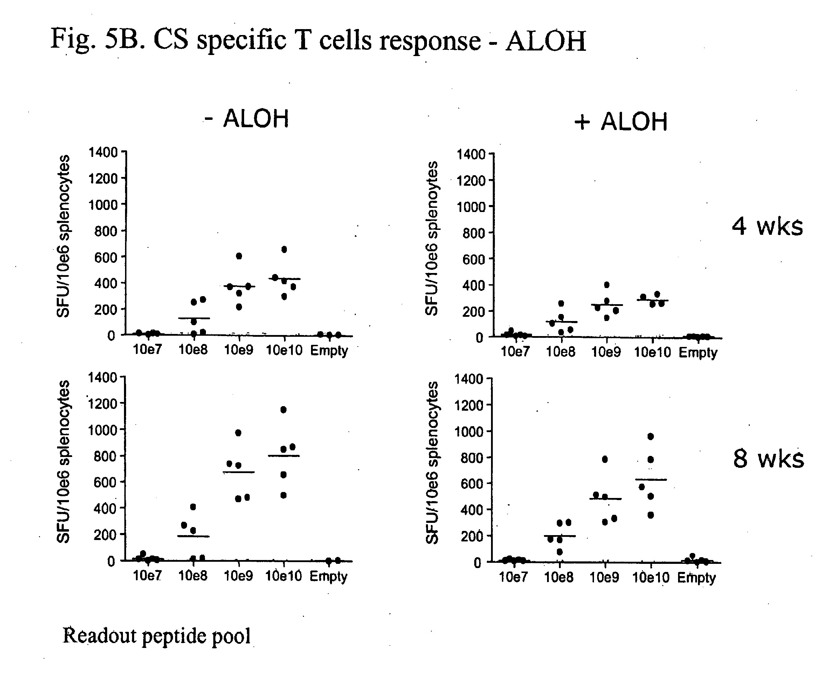

Before injection, solutions were incubated for 30 min at 4°C by rotation and prepared typically as described above. The mice were immunized with 50 $\mu$l of virus vaccine in the quadriceps of both hind legs (100 $\mu$l per mouse). At week 4 and week 8 mice were sacrificed and CS specific cellular immune responses were determined by gamma-interferon (IFN-$\gamma$) ELISPOT assays as previously described (Barouch DH et al. 2004. J. Immunol 172:6290-6297). Murine splenocytes were assessed for responses using a CS peptide from a dominant epitope: NYDNAGTNL (SEQ ID NO:4) (H-2K$^d$). For the differences between the absence and presence of aluminium hydroxide, results are shown in Figure 5A. The presence of Aluminium hydroxide in the vaccine composition (right upper graph) did not improve the anti-CS T-cell response at week 4, as compared to rAd35-CS vaccine immunizations in the absence of Aluminium hydroxide (left upper graph; p=0.71, ANOVA). Similar results were obtained at 8 weeks after immunization (right lower graph versus left lower graph; p=0.75, ANOVA). These data were confirmed using a pool of overlapping peptides together covering the entire CS protein (Figure 5B).

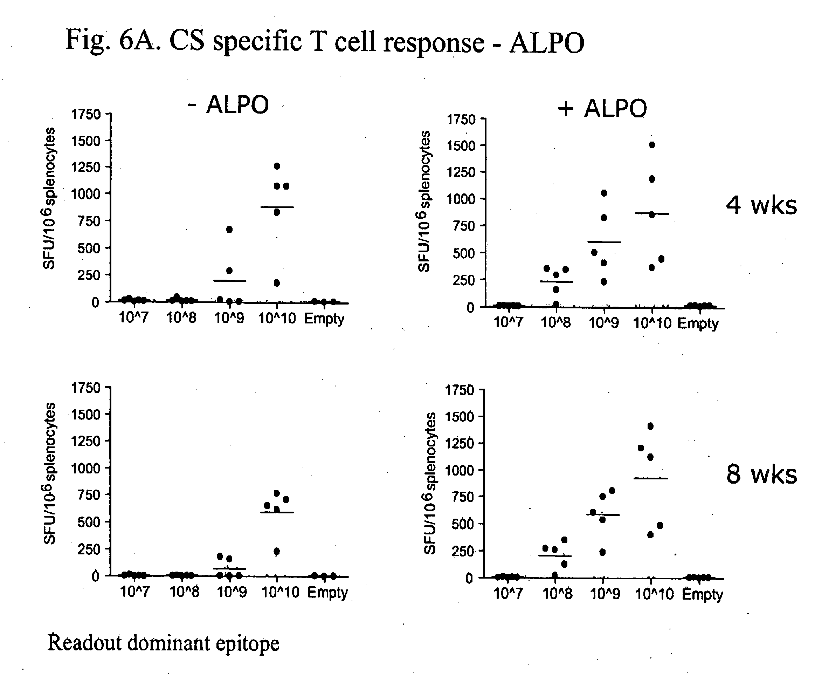

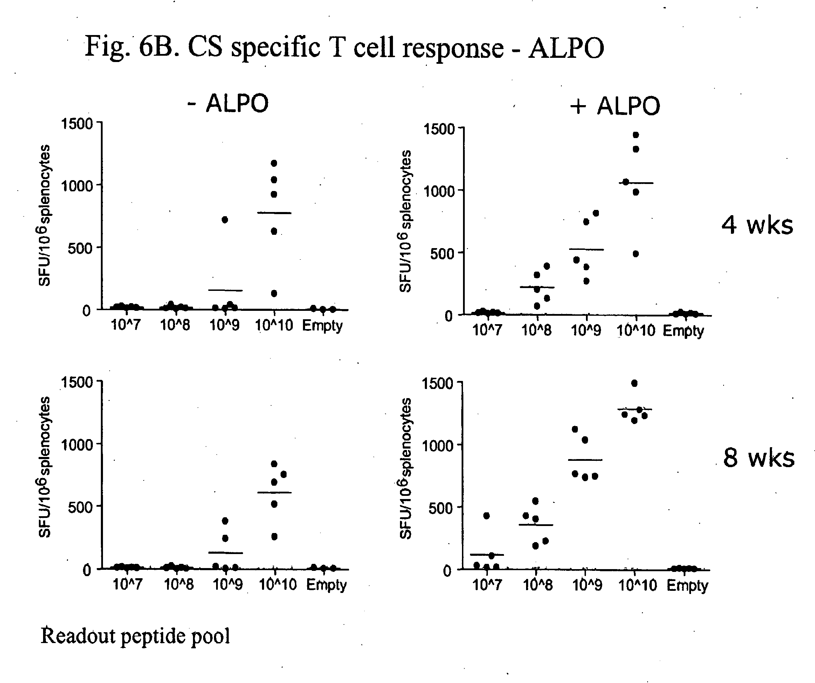

The same experiment was performed with aluminium phosphate. Notably, in contrast to what was found with aluminium hydroxide, the presence of aluminium phosphate did significantly (p= 0.002 ANOVA) improve the week 4 anti-CS T-cell response as determined using the dominant epitope-containing peptide as compared to rAd35-CS vaccine immunizations in the absence of aluminium phosphate (compare Figure 6A left and right upper panels). Data obtained at week 8 after rAd35-CS immunization coincided with the week 4 data demonstrating significant increases in anti-CS T-cell responses (p < 0.001 ANOVA; Figure 6A, right lower graph versus left lower graph). These data were again confirmed using a pool of overlapping peptides together covering the entire CS protein (Figure 6B).

These results show that it is possible to increase the cellular immune response towards an immunogenic determinant encoded by a gene, which is incorporated into the genome of a recombinant adenovirus, when this adenovirus is brought in one composition with an aluminium-based adjuvant. Clearly, the aluminium-phosphate adjuvant is preferred, as indicated by the results obtained with Aluminium phosphate.

## Example 4. Effect of an aluminium-based adjuvant on anti-insert specific antibody responses *in vivo*

As described in example 3 above, another group of BALB/c mice (n=5 per time point) were immunized with 2 x 50$\mu$l of a composition comprising recombinant adenovirus based on Ad35, carrying the CS transgene, including or excluding an aluminium-based adjuvant. The injections were performed in the quadriceps of both hind legs (50 $\mu$l per leg).

At week 0, 2, 4, 6, and 8, blood samples were taken and sera were harvested to determine the presence of antibodies that were able to recognise the CS protein. An ELISA was used as described (Ophorst OJ et al (2007) Vaccine 25:1426-36). Hereto, maxisorp ELISA plates (Nunc) were coated with 2 μg/ml CS specific peptide. This peptide comprises six stretches of a NANP sequence followed by a Cystein residue (denoted as $(NANP)_6C$). The peptide was dissolved in 0.05 M Carbonate buffer. Plates were washed with PBS/0.1% Tween-20 and blocked with 200 μl PBS/1.0% BSA/0.05% Tween-20 for 1 h at 37°C.

Plates were washed again and then 100 μl serially (two-fold) diluted sera in PBS/0.2% BSA/0.05% Tween-20 was added to the wells and incubated for 2 h at 37°C. The plates were subsequently washed and incubated with 100 μl IgG Biotin (DAKO), 1:1000 in sample buffer for 30 min at 37°C. Finally, plates were washed and 100 μl per well of Phenylenediamine dihydrochloride (OPD, Sigma) substrate was added to each well after which plates were incubated for 10 min at room temperature in a dark environment. The optical density was measured at 492 nm.

The adenovirus antibody neutralization assay was performed in 96-wells flat-bottom micro-titer plates as previously described (Sprangers MC et al (2003) J Clin Microbiol 41:5046-52). Briefly, a twofold dilution of sera was prepared, starting from a serum dilution of 1/32. To this mixture, $5x10^6$ vp of recombinant adenovirus containing the luciferase reporter gene (rAd35Luc) in a volume of 50 μl was added followed by the addition of 100 μl medium containing $10^4$ A549 cells (multiplicity of infection of 500). Luciferase reporter gene expression in cells was assessed using luciferase substrate and Trilux luminescence detector (according to the manufacturer's instruction). Data was analyzed using non-linear regression to calculate the antibody inhibitory concentrations of 90% (IC90) in the sera samples.

Statistical analysis

The results on all variables have been analyzed by fitting logistic sigmoid curves on the $Log_{10}$ values of the results (Eq.1). This model was selected because it gives robust estimates for all the observed characteristics of the conducted experiments.

$$Log_{10}(Y) = A + B(Expt.) \times \frac{10^{**}(f(X))}{1 + 10^{**}(f(X))} \qquad (1)$$

A= the background noise level of the results (left/lower asymptote)
B(Expt.) = the saturation level of the results, depending on the experiment (right/upper asymptote), and
$f(X)$ = the linear function of the effect with Dose and the investigated treatments (Eq.2).
$Log_{10}(Y)$ are the Log base 10 transformed values of the measurements.

$$f(X) = c_0 + c_1 \times (Log_{10}(Dose) - Treatment.Effect) \qquad (2)$$

$c_0$ and $c_1$ are the regression coefficients of the linear model (the intercept and slope, respectively).
$^{10}Lg(Dose)$ are the provided values for the Dose treatment.
The Treatment.effect equals the horizontal shift between the curves with and without $AlPO_4$.
The value for $c_0$ was set to 0 and the treatment effect was estimated for both curves, so that the shift between the curves was estimated at the point of inflection (Y=0 on the linear scale).

The lower (left) asymptotic reflects the background noise. The upper (right) asymptotic reflects the saturation level. This value was made experiment-dependent, since the level of saturation may vary considerable between experiments. The effect of the treatments (aluminium phosphate) was estimated as the horizontal shift on the linear scale. The obtained values are equal to the horizontal shift between the points of inflection at the original scale.

Data are presented as (geo) means. Statistical analyses were performed with SPSS version 12.0.1 (SPSS Software, Inc., 2004). Immune responses (logarithmically transformed) among groups of animals were assessed with UNIANOVA or student T-test. Differences were considered significant when p<0.05.

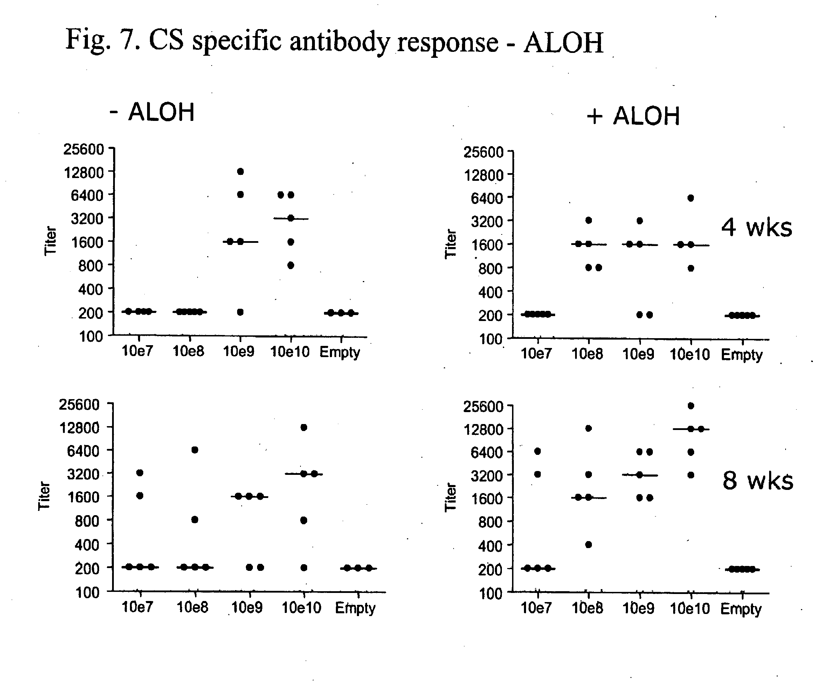

The results obtained from the anti-CS specific ELISA using sera derived from mice that received either rAd35-CS vaccine alone or mixed with aluminium hydroxide are depicted in Figure 7. The presence of aluminium hydroxide (right upper graph) did not significantly provide an increasing adjuvant effect in the $10^9$ and $10^{10}$ vp dosages, as seen with the week 4 anti-CS antibody response in comparison to rAd35-CS vaccine immunizations in the absence of aluminium hydroxide (p > 0.05, Wilcoxon, left). Notably, applying aluminium hydroxide at the dose of $10^8$ vp, an increased humoral immune response was detected (p = 0.008). No significant differences were observed at week 8 after immunization (p > 0.05, Wilcoxon, Figure 7, lower panels).

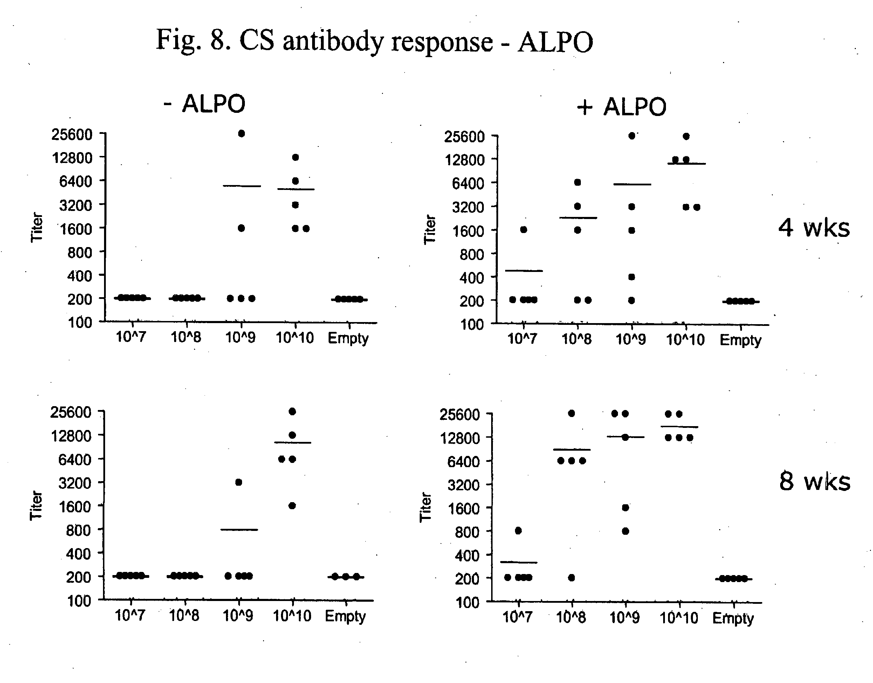

The same experiments were performed with aluminium phosphate, which resulted in a marked increase of antibody titers in comparison to what was found using aluminium hydroxide, when the antibody response was measured at the 8-weeks time point. The results obtained from the anti-CS specific ELISA using sera derived from mice that received either rAd35-CS vaccine alone or mixed with aluminium phosphate are depicted in Figure 8. Although with the low doses of recombinant virus ($10^7$ and $10^8$ vp) an increase was seen in the presence of Aluminium phosphate at week 4, the higher doses did not show an increase in response (p > 0.05). However, significant increases were observed at week 8 after immunization, and in particular for the doses of $10^8$ and $10^9$ virus particles (p = 0.032, Wilcoxon, Figure 8, lower panels). This lag period in which antibody titers rise over time is not yet fully understood, but nevertheless it has been shown that (taking the results from Example 3 along), a significant increase in humoral as well as cellular immune response can be obtained using a recombinant adenoviral vector in combination with an aluminium-based vaccine, especially with respect to aluminium phosphate. Importantly, when the antibody responses are plotted against the dose, it appears that in order to obtain a response (with a composition lacking the adjuvant) that is comparable to a response obtained with a composition including aluminium phosphate, a 10x higher dose is required, whereas the antibody response towards the hexbn protein of the adenoviral capsid differs only 0.5 log (data not shown). Similar results were obtained with respect to the T cell response towards the CS antigen. Together this is a clear indication that lower doses may be used for vaccination in order to obtain the same proper level of immunization, which may be even tenfold lower. Aluminium phosphate is therefore considered a strong and very useful immunopotentiating compound when applied together with a recombinant adenoviral vector, based on Ad35, wherein the viral vector preferably carries a nucleic acid encoding the CS protein of a malaria-causing pathogen, when subjects need to be vaccinated against malaria.

**Example 5: Effect of aluminium-based adjuvant on anti rAd35 vector specific cellular and humoral responses in vivo.**

Next to determining the anti-CS specific immune responses in absence or presence of aluminium-based adjuvant preparations (see above), it was also determined whether there was any effect of these adjuvant-containing preparations on immunity against the adenoviral particle itself.

Cellular immune responses against the recombinant Ad35 carrier were determined by a IFN-$\gamma$ ELISPOT assay using a peptide located in the rAd35 hexon capsid protein, which peptide covered a dominant CD8 epitope in BALB/C mice (sequence: KYTPSNVTL; SEQ ID NO:3), assessing the murine splenocytes. The ELISPOT assay was typically performed using general methods known to the skilled person.

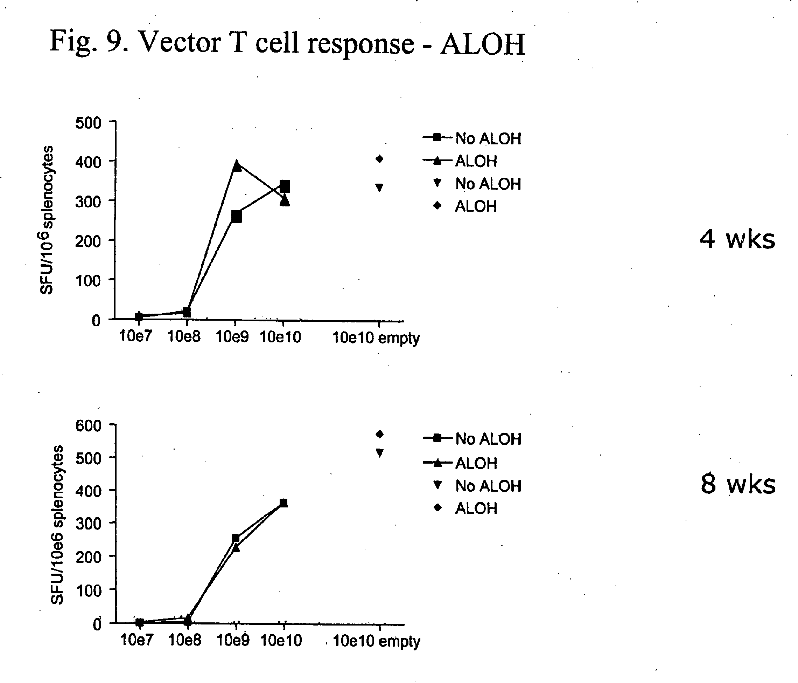

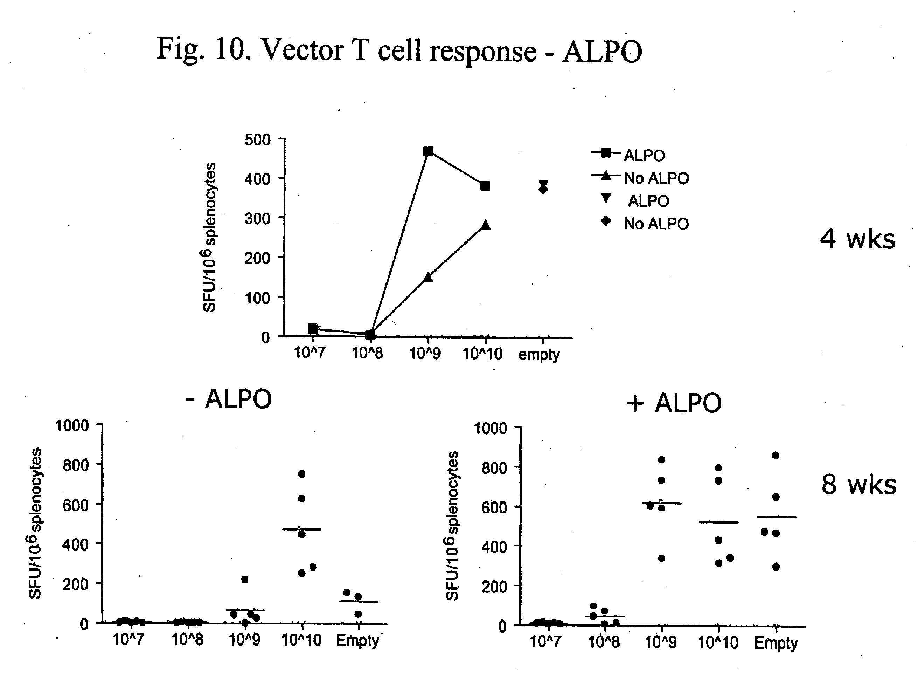

As shown in Figure 9, the presence of aluminium hydroxide did not result in an increase in T-cell responses against the rAd35 carrier at either 4 weeks (upper panel) or 8 weeks after rAd35-CS vaccine immunization (lower panel). In contrast, using Aluminium phosphate, an increase in anti-rAd35 T-cell response was observed at week 4 (Figure 10, upper graph). This enhanced anti-rAd35 T-cell response in the presence of Aluminium phosphate was sustained at week 8 after immunization (Figure 10, lower panels).

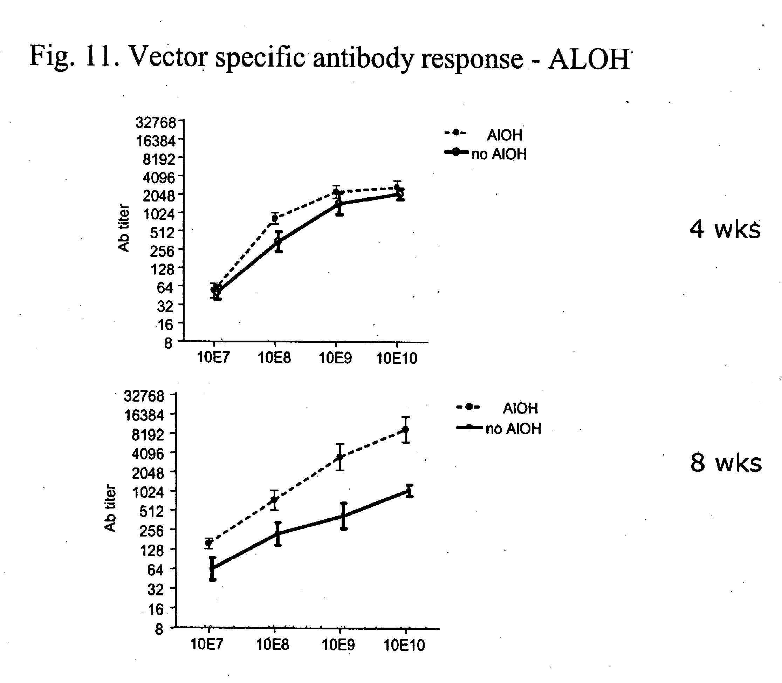

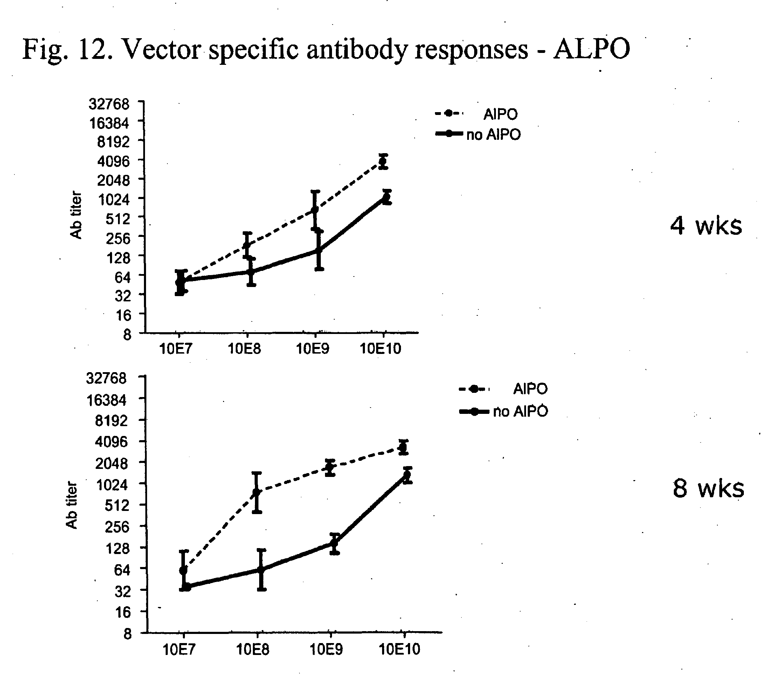

Anti-recombinant Ad35 antibodies were determined using a method that was described earlier (Sprangers MC et al. 2003. J Clin Microbiol 41:5046-5052). The results are shown in Figure 11. It shows that antibody levels against the recombinant Ad35 particle were elevated in mice both at week 4 (upper panel; p = 0.53, ANOVA) and week 8 (lower panel; p < 0.01, ANOVA) after injection of a composition comprising aluminium hydroxide in comparison to injections lacking the adjuvant. The effects seen at week 8 were more visible than at week 4. This shows that, although the aluminium hydroxide does not provide an increase in cellular immune responses, it is able to increase an antibody response, in this case towards the viral particle itself. Similar results were obtained with compositions comprising or lacking the other aluminium-based adjuvant, aluminium phosphate (see Figure 12): antibody levels against the rAd35 carrier are elevated both at week 4 (upper graph; p = 0.009, ANOVA) and at week 8 (lower graph; p < 0.0001, ANOVA). However, when the response against the Ad35 particle itself is calculated against the response towards the antigen, it turns out that the increase in neutralizing activity (directed against the hexon protein in the capsid) is lower than the increase in immune response against the antigenic determinant, indicating that the benefit with respect to the antigen prevails over the negative effect against the viral particle itself.

It was also tested whether a different dose of adjuvant would influence the effect on the CS antigen and/or on the vector particle. Effects were assessed at 4 and 8 weeks after injection of a $10^8$ vp dose of Ad35.CS with or without an aluminium based adjuvant (aluminium hydroxide and aluminium phosphate) in three different doses: 0.5, 2.0 and 4.0 mg/ml. No effect of the different dosages was observed in this regimen on neutralizing antibodies or on antibodies and T-cell responses towards the CS antigen (results not shown).

It is concluded that the aluminium phosphate adjuvant increases the antibody and T-cell response towards the CS antigen carried by a recombinant replication-defective adenovirus based on serotype 35. The immune response seen with aluminium phosphate relates to a significant increase of 0.5-1.0 log in relation to the CS antigen.

**Example 6. Effect of oil-in-water based adjuvants on anti insert specific T-cell responses *in vivo***

The same experiments as outlined in the examples above were performed with two oil emulsion-based vaccine adjuvant compounds, Covaccine HT and MF59, instead of the aluminium-based adjuvants.

Mice (BALB/C, 6 weeks old purchased from Harlan) were injected with a recombinant Ad35 virus carrying the CS cDNA. The rAd35-CS virus was dissolved in TRIS buffer using different vaccine doses ($2x10^7$, $2x10^8$, $2x10^9$ and $2x10^{10}$ vp/100 $\mu$l TRIS buffer). Dilutions were divided in two tubes and mixed with equal volumes (1:1) of TRIS buffer of either one of the mentioned oil-in-water adjuvant compounds (Covaccine HT and MF59). The end concentration of the adjuvant in the composition to be injected was 1 v/v and for the recombinant Ad35-CS $10^7$, $10^8$, $10^9$ and $10^{10}$ vp/100 $\mu$l. An empty recombinant Ad35 virus in a concentration of $10^{10}$ vp was used as a control.

Mixtures were incubated for 30 min at 4° C by rotation before immunization. 10 BALB/c mice per group were immunized with 50 $\mu$l of vaccine in the quadriceps of both hind legs (100 $\mu$l per mouse). At week 4, 5 mice were sacrificed and at week 8 after injection 5 mice were sacrificed from each group, and subsequently CS specific cellular immune responses were determined by IFN-$\gamma$ ELISPOT assays as described above.

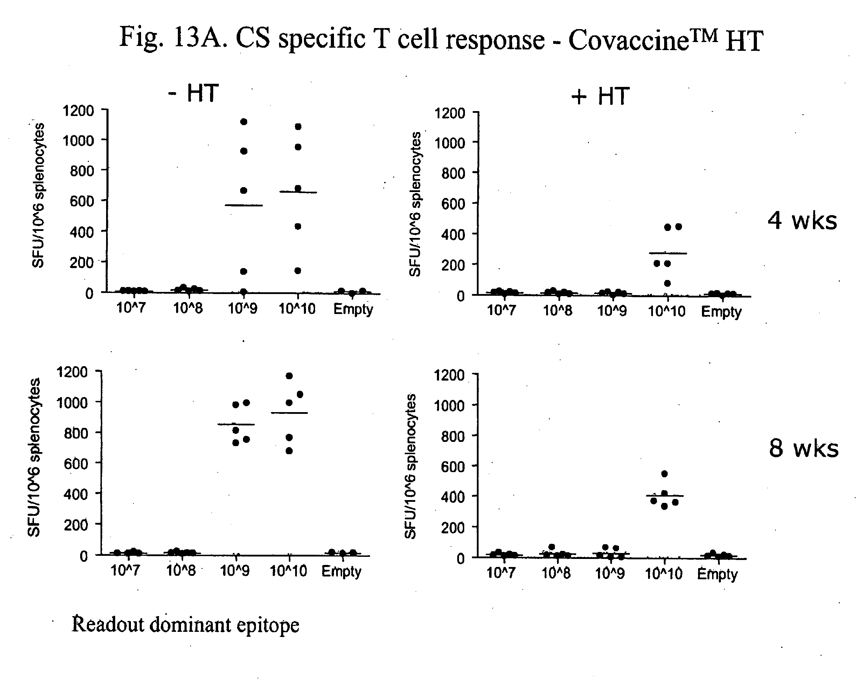

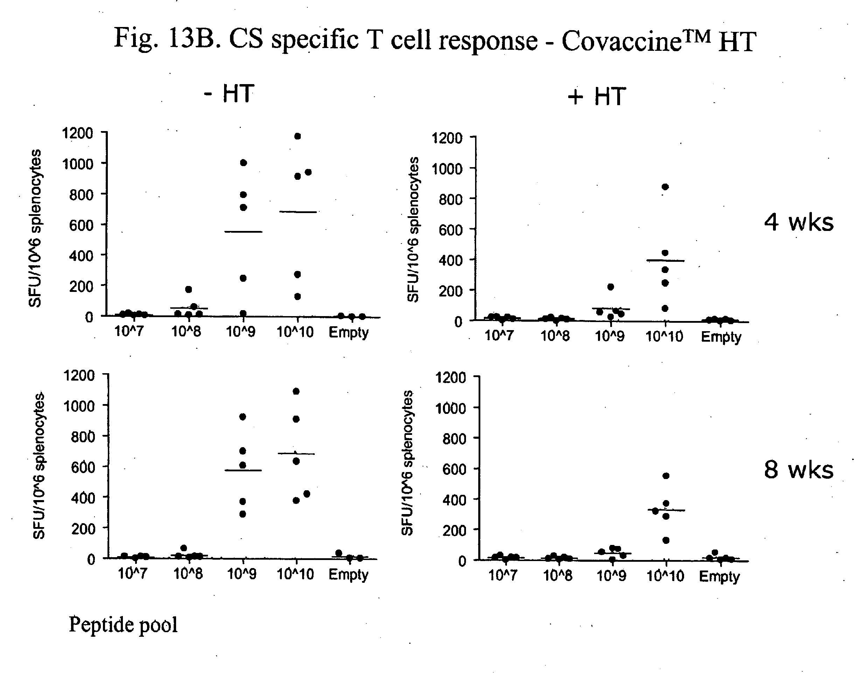

The results with the Covaccine HT adjuvant are shown in Figure 13 (A refers to results using the dominant epitope, whereas B refers to results using the peptide pool covering the CS protein). Importantly, no increase in cellular immune response was seen using this adjuvant; responses were even higher when no adjuvants was used, most likely due to possible toxic effects on cellular viability.

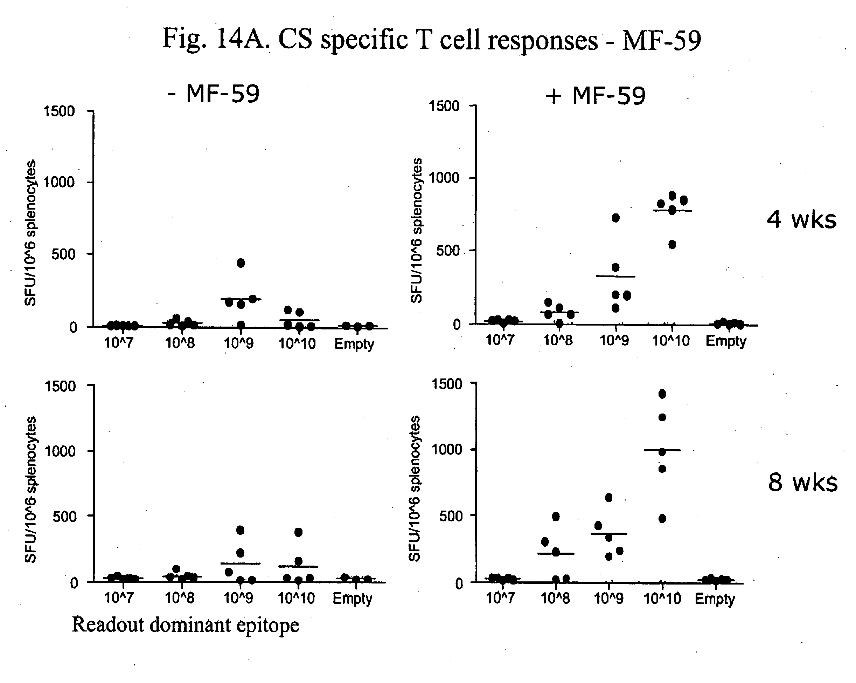

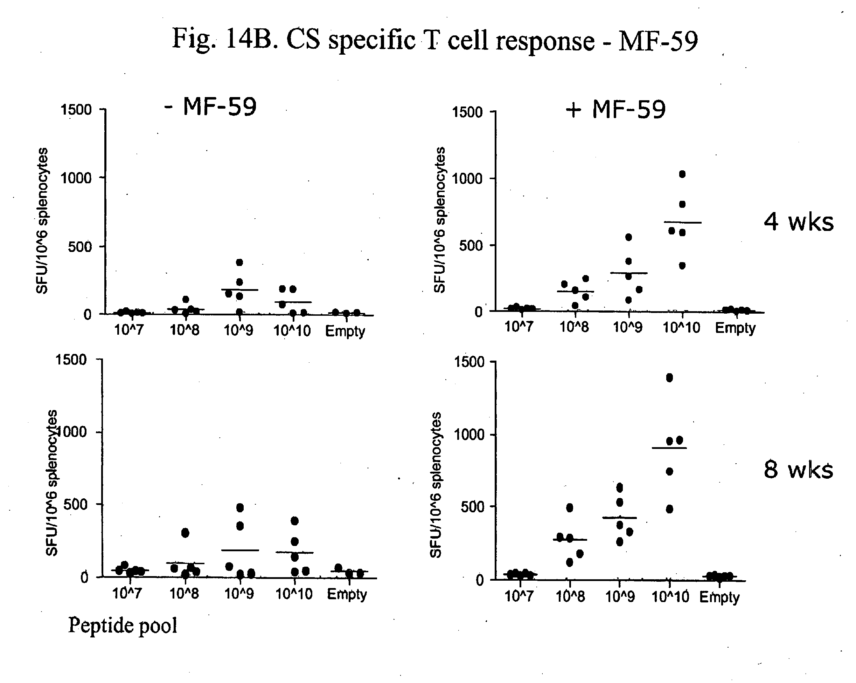

The results with the MF59 adjuvant are shown in Figure 14. Here, surprisingly, in contrast to what was found with the Covaccine HT compound, an important and significant ($p < 0.001$) increase in cellular immune response was detected, both in the 4 week as well as the 8 week samples, either using the dominant epitope (Figure 14A) or the antigen-spanning peptide pool (Figure 14B), to assess these murine splenocytes.

The conclusion is that MF59 is an excellent adjuvant to use in vaccine compositions comprising recombinant adenoviral vectors, as it is able to significantly stimulate cellular immune responses towards the immunogenic determinant encoded by the heterologous gene incorporated in the adenoviral genome.

**Example 7. Effect of oil-in-water based adjuvants on anti insert specific antibody responses *in vivo***

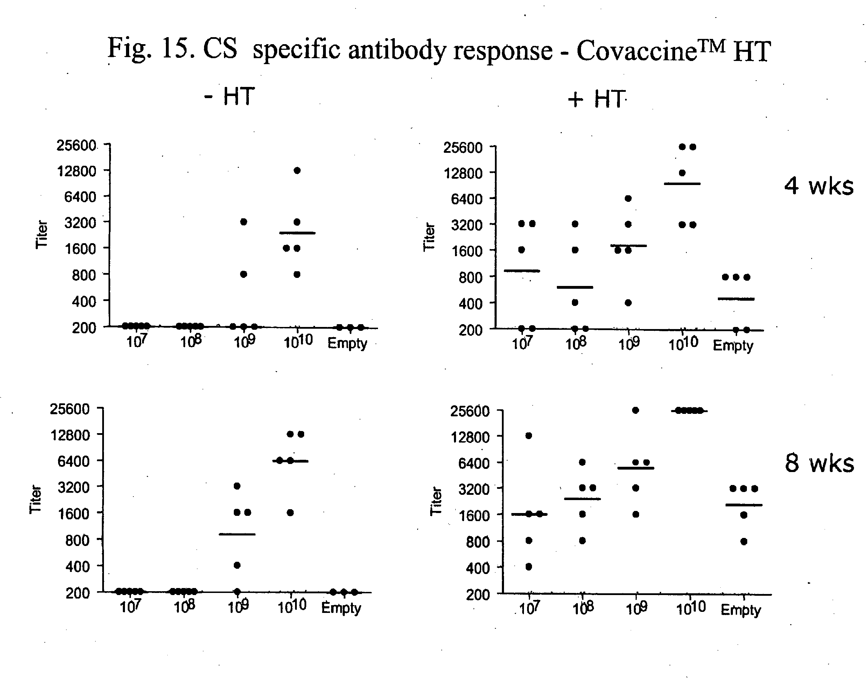

In line with what was studied and outlined in Example 4, it was further investigated whether the two examples of the oil-emulsion adjuvants (Covaccine HT and MF59) also could contribute to the antibody response towards the protein encoded by the heterologous transgene, herein exemplified by the CS protein. The mice that were used as described in Example 6 were also used to provide blood samples at week 0, 2, 4, 6, and 8. Sera were harvested to determine the presence of antibodies that recognise CS. These studies were typically performed as outlined in Example 4. The results on the antibody response towards CS upon injection with recombinant Ad35-CS viruses with or without Covaccine HT are shown in Figure 15. Interestingly, although hardly any effect was determined with respect to the cellular immune responses (see Figure 13), a significant increase was determined with regard to the antibody response. Especially at the 8 week time point, all doses gave a significant boost in antibody response when applied in combination with Covaccine HT ($p < 0.05$, Wilcoxon).

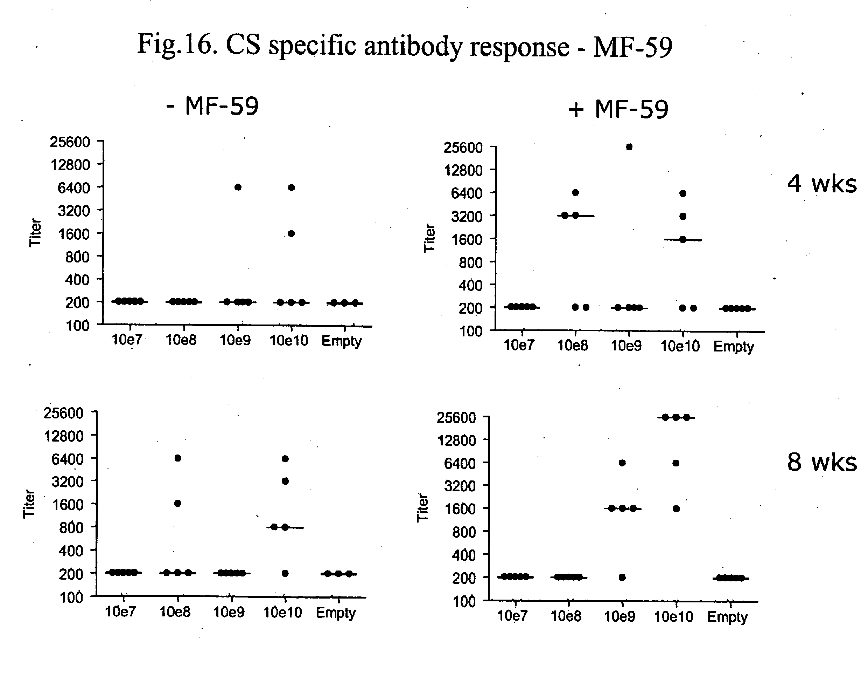

It is concluded that although the oil-emulsion adjuvant compound Covaccine HT may not be useful in inducing a T cell response in a vaccine together with a recombinant adenovirus, it may be very useful in stimulating antibody responses. Care has to be taken with respect to the possible toxicity that was observed with this compound (in a composition with recombinant adenovirus), but since all different adenoviral doses gave an increased response in the context of Covaccine HT, a particular suitable dose maybe sought that is not toxic and that may still benefit from the antibody-stimulating effects of this oil-emulsion adjuvant. The same experiments were executed with the other example of the class of oil-emulsion adjuvants, MF59. These experiments and measurements were typically performed as described above. The results on the antibody response using this particular compound in a combination composition with recombinant Ad35-CS is shown in Figure 16.

The results demonstrate that at week 4, the presence of MF59 (right upper panel) did not significantly improve antibody response against CS in comparison to immunization with recombinant Ad35 virus alone (left upper panel; $p > 0.05$, Wilcoxon). At week 8 a significant increase in CS-specific antibody responses was observed, in particular at a recombinant Ad35 virus dose of $10^9$ and $10^{10}$ virus particles ($p = 0.032$, Wilcoxon).

It is concluded that both Covaccine HT and MF59 are suitable adjuvants that may be used in vaccine composition comprising recombinant adenoviruses, especially when antibody responses are sought. With respect to cellular immune response, it turns out that MF59 is preferred as this compound significantly contributed to an increased T cell response towards the antigen encoded by the transgene carried by the recombinant adenovirus.

**Example 8. Effect of oil-in-water based adjuvants on anti rAd35 vector specific cellular and humoral responses *in vivo***

As with the aluminium-based adjuvants, it was also investigated whether the adjuvants had an effect on the immune responses towards the viral particle present in the composition. These experiments were typically performed as described above in Example 5.

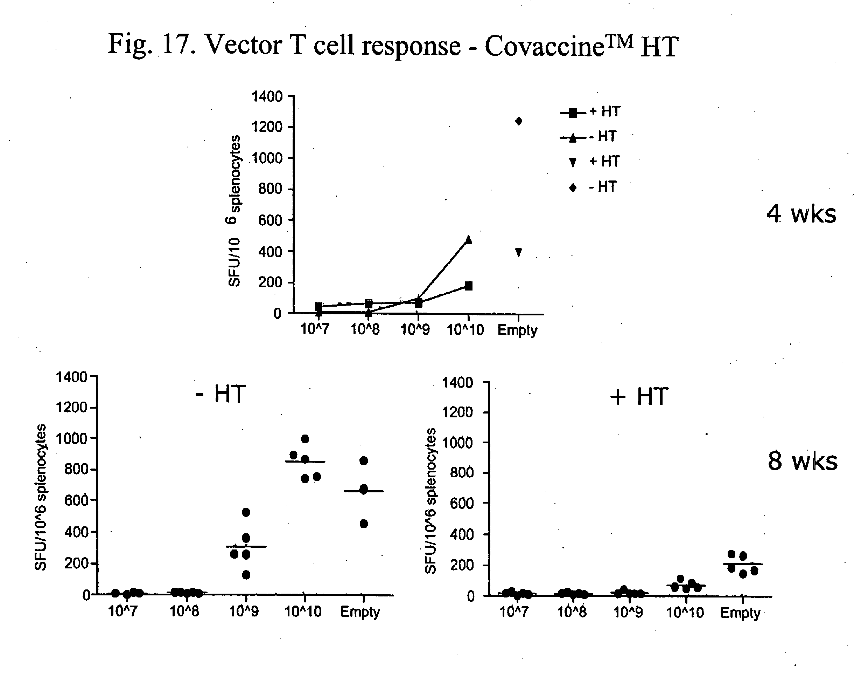

Figure 17 shows the results on the T cell responses towards the vector using Covaccine HT. Interestingly, in the presence of Covaccine HT, there is actually a significant decrease in cellular immune response towards the vector at the 4 weeks time point as well as at the 8 weeks time point. This effect may actually be beneficial in settings wherein the immune response against the insert is unwanted, and wherein the immune response against the gene delivery vehicle is also unwanted, as this may lower its efficacy. Such settings are mainly in the field of gene therapy, where the gene of interest needs to be brought into the host cell with an immune response that is as low as possible, and where the immune response against the vector should also be low. From the results shown in Figure 17 it is concluded that Covaccine HT is a good candidate to lower the immune response towards the gene delivery vehicle, making this a potentially beneficial compound to be used in gene therapy settings.

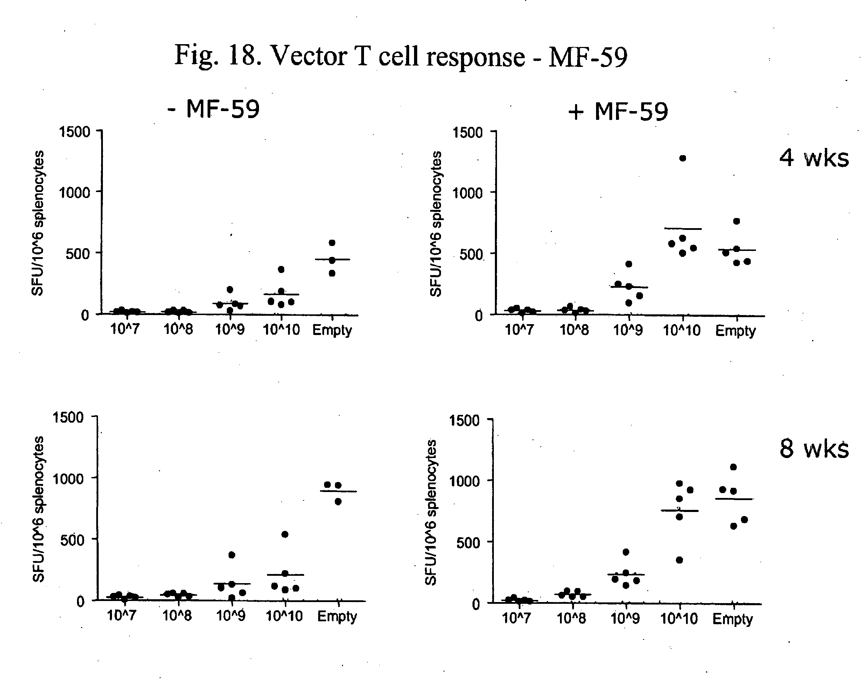

In contrast to Covaccine HT, MF59 does stimulate the immune response towards the viral vector, as can be seen in Figure 18.

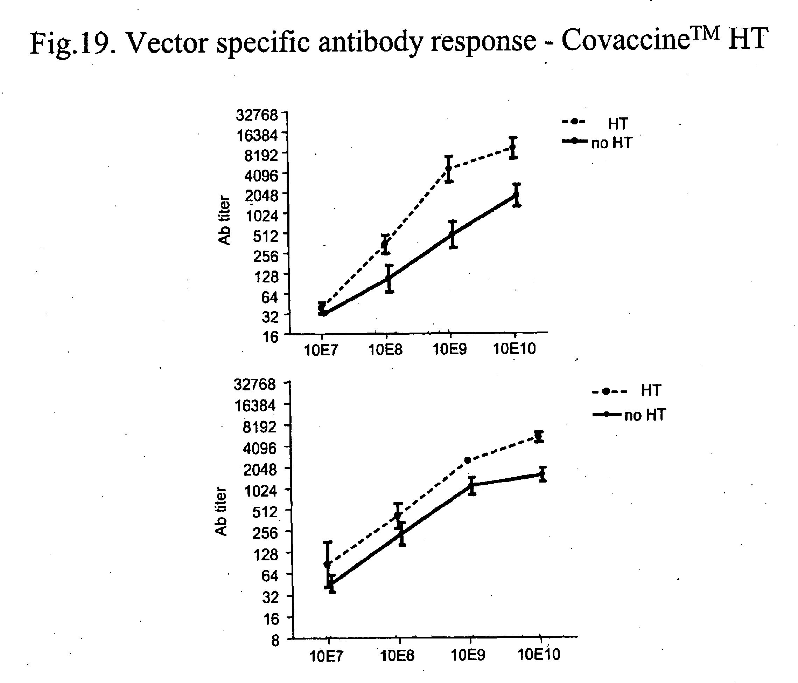

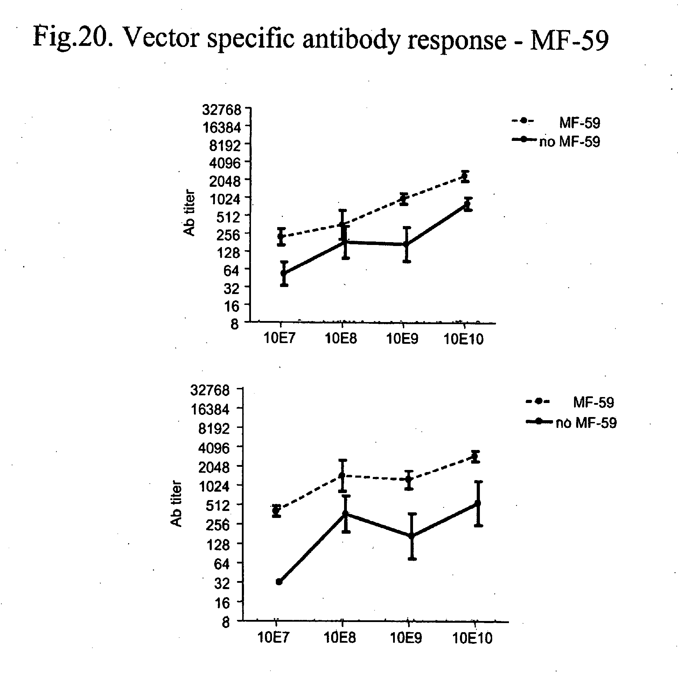

In line with the antibody responses seen towards the CS protein encoded by the heterologous insert in the adenoviral genome, but in contrast to the cellular immune response, Covaccine HT does increase the antibody response towards the viral vector (see Figure 19). Also the addition of MF59 gave such similar results (see Figure 20).

It can be concluded that, depending on the purpose and the immune response needed, with respect to oil-emulsion adjuvants, one could choose between Covaccine HT and MF59, whereas it was found that for both cellular and humoral immune responses it is preferred to use Aluminium phosphate instead of Aluminium hydroxide when aluminium-based adjuvants are to be applied.

**Example 9. General applicability of aluminium phosphate in adenovirus-based vaccines**

From the data provided above, it becomes clear that a recombinant adenoviral vector that is based on serotype 35 benefits from the addition of an adjuvant, since the antibody and cellular response towards the antigen is increased. It is also shown that not all adjuvants provide such a positive effect, but that the preferred adjuvant is an aluminium-phosphate adjuvant. To address whether the applicability of aluminium phosphate in the context of an adenoviral vector could be found with other serotypes as well, it was tested whether adenoviral vectors based on two other serotypes could also benefit from the addition of this adjuvant. Also, a number of other antigens were assessed. Two other vectors were compared to Ad35, namely a recombinant replication-defective Ad5 vector (subgroup C) and a recombinant replication-defective Ad49 vector (subgroup D). Two other antigens were selected: The Simian Immunodeficiency Virus Gag antigen (SIVGag) and a chimeric simian/human immunodeficiency virus (SHIV) Env antigen (SHIVEnv). The following viruses were used in these studies: Ad35.SIVGag, Ad5.SIVGag, Ad49.SIVGag, Ad35.SHIVEnv, Ad5.SHIVEnv and Ad49.SHIVEnv, which vectors were constructed and produced in line with the vectors discussed above (Ad5.SIVGag and Ad35.SIVGag: Barouch DH et al (2004) J Immunol 172:6290-7*; Ad49.SIVGag: Lemckert AAC et al (2006) J Gen Virol 87: 2891-9)*. The SHIVEnv viruses were constructed accordingly and contain a chimeric Env antigen described by Reimann KA et al (1996) J Virol 70:6922-8.

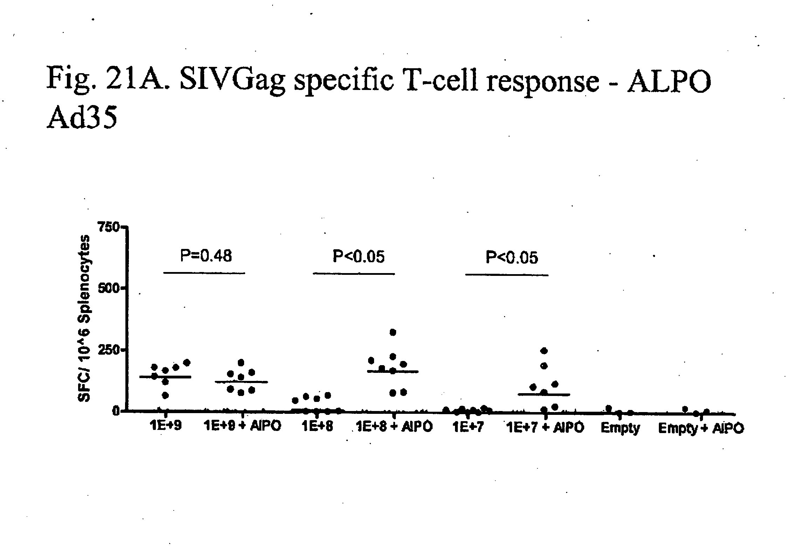

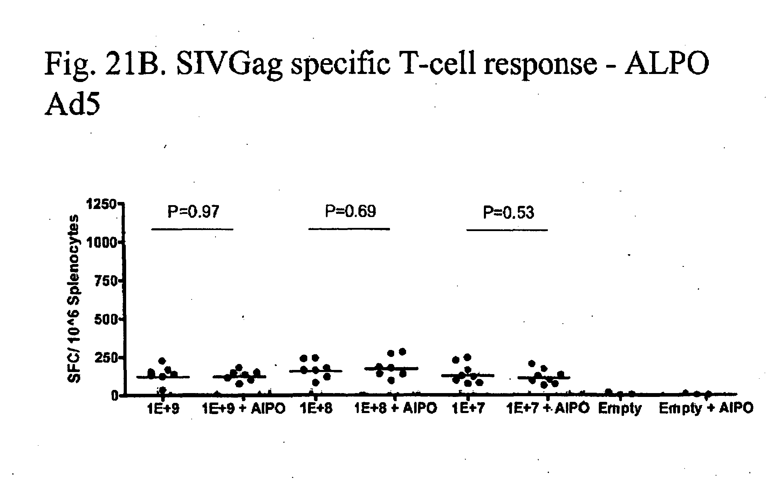

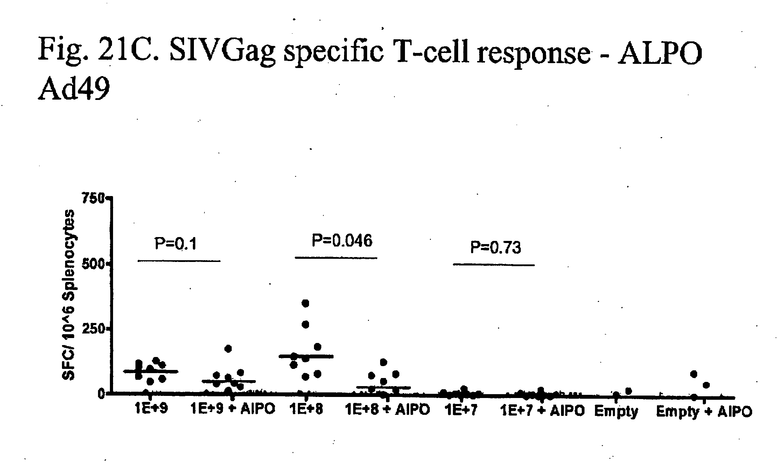

First, the T-cell response towards the SIVGag antigen was measured from the three different vectors in the presence or absence of aluminium phosphate. For this, a total of 54 Balb/C mice were included in a study using $10^7$, $10^8$ and $10^9$ vp of each vector with and without adjuvant. Elispot assays were performed 8 weeks after injection. Figure 21A shows the effect of adding aluminium phosphate to the three different doses using the Ad35.SIVGag virus. Clearly, a statistically significant increasing effect is observed with the two lower doses $10^7$ and $10^8$ vp. In contrast, when one uses the Ad5.SIVGag virus, no effect is observed, see Figure 21B. This is also true when Ad49.SIVGag is injected. Although the effect is not statistically significant, there even seems to be a negative trend by applying this adjuvant, see Figure 21C.

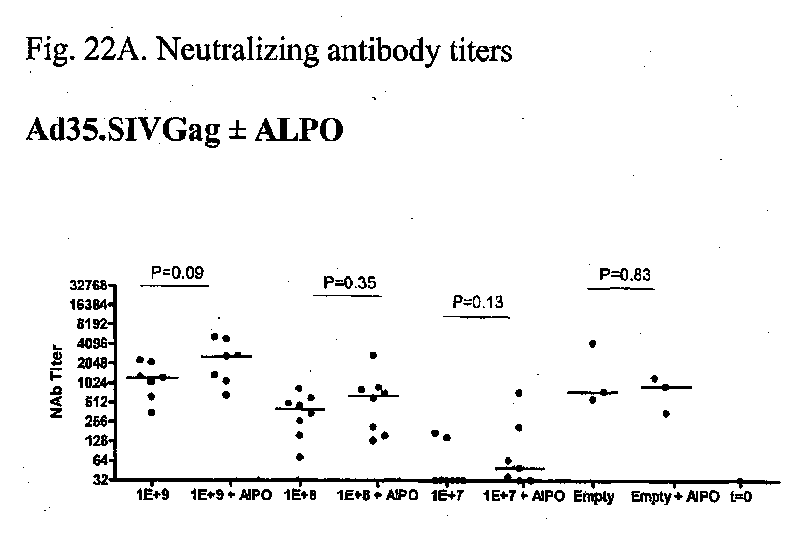

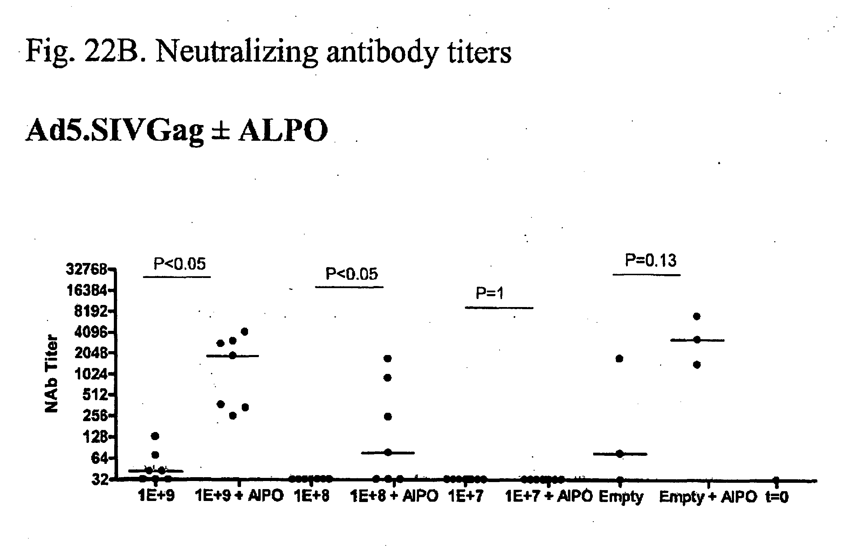

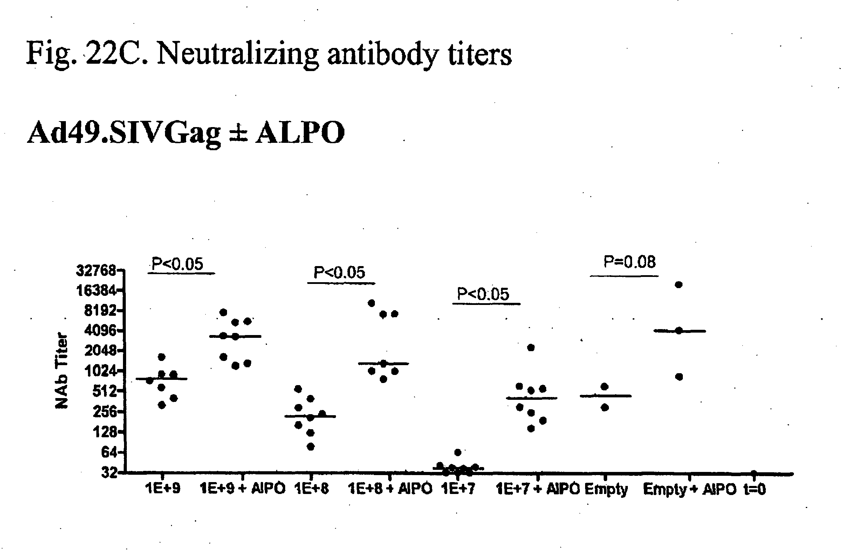

The antibody response towards the different vector capsids were also measured, generally represented by neutralizing antibodies (NAbs) against the hexon protein. When one applies Ad35.SIVGag in different doses with or without aluminium phosphate, no statistically significant increase or decrease of NAb titers was found, see Figure 22A. However, when Ad5.SIVGag or Ad49.SIVGag were applied, a significant increase in NAb titers was found with different doses, see Figure 22B and Figure 22C respectively. From these experiments it is concluded that there is a strong serotype-specific response when applying recombinant adenovirus vectors harboring an antigen in the context of aluminium phosphate adjuvant. The preferred adenovirus serotype is Ad35, while, based on these studies, Ad49 in a composition with aluminium phosphate is not preferred; at least not when the SIVGag antigen-encoding gene is incorporated in the vector genome.

The T-cell response against SIVGag was also compared with the T-cell response against another SIV antigen:

SHIVEnv. To develop an improved AIDS animal model, chimeric simian/human immunodeficiency viruses (SHIVs) composed of SIVmac239 expressing HIV-1 env and the associated auxiliary HIV-1 genes tat, vpu, and rev were constructed and used to infect rhesus monkeys. After two serial *in vivo* passages by intravenous blood inoculation of naive rhesus monkeys, a virus was obtained that displayed an AIDS-like disease (SHIV-89.6P; Reimann KA et al (1996) J Virol 70:6922-8). Here, the Env gene derived from this SHIV virus was used to construct the adenoviral vectors Ad35.SHIVEnv, Ad5.SHIVEnv and Ad49.SHIVEnv. The precursor envelope (Env) polyprotein is called gp160 and includes the glycoproteins gp120 and gp41. The glycoprotein gp120 is exposed on the surface of the viral envelope and binds to CD4. The gp41 is non-covalently bound to gp120 and is situated within the viral envelope (transmembrane glycoprotein). A truncated form of gp160 was used in the experiments discussed herein, resulting in the expression of only gp120.

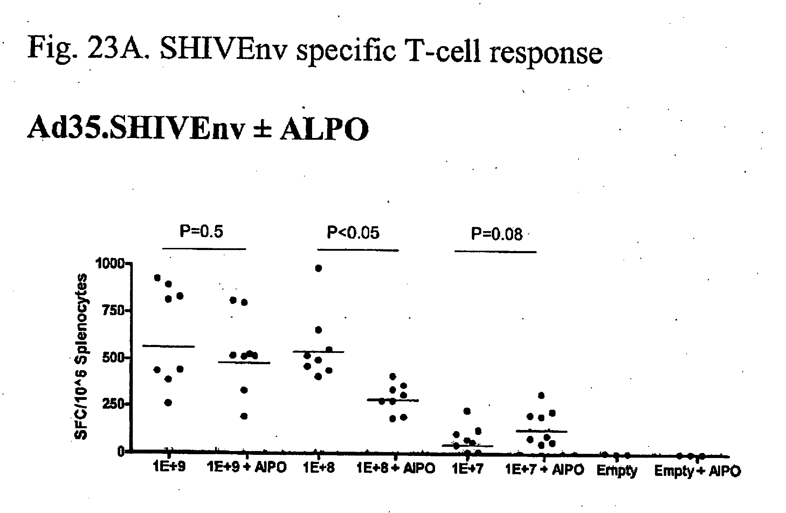

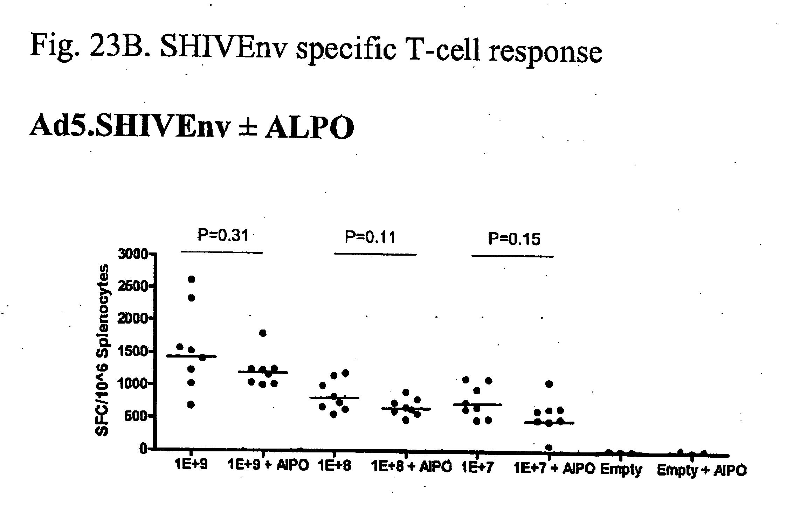

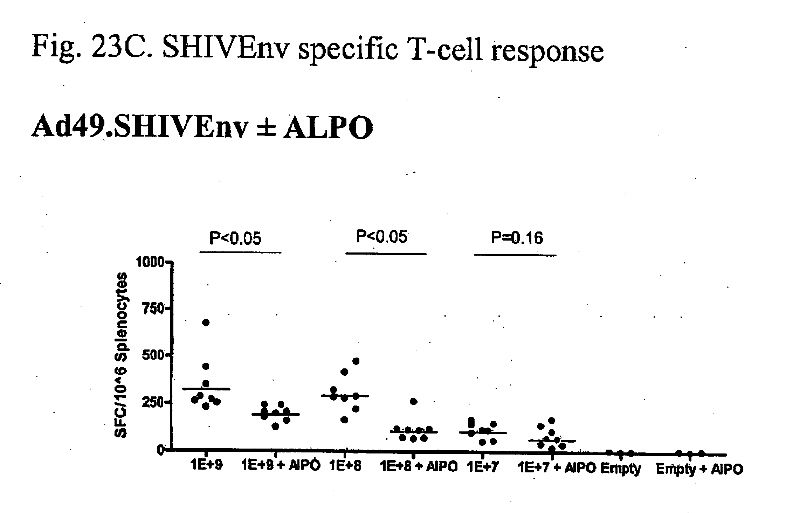

From Figure 21A (see above) it already became clear that a positive effect could be observed when applying the two lower doses of Ad35.SIVGag together with aluminium phosphate, indicating a positive effect towards any antigen carried by a recombinant Ad35 virus in the presence of this aluminium-based adjuvant. When SHIVEnv was used (Ad35.SHIVEnv), no change in neutralizing antibody titers towards the vector could be determined (Figure 22A), whereas these titers were significantly higher in the case of Ad5-and Ad49 based vectors (Figures 22B and 22C), which indicates that Ad35 is again preferred: The neutralizing activity towards the viral vector capsid itself is not substantially influenced by adding aluminium phosphate. Notably, when $10^8$ vp of Ad35.SHIVEnv was used together with aluminium phosphate, a decrease in T-cell response was measured, which was not apparent with the two other doses ($10^7$ and $10^9$ vp), see Figure 23A. No effect on T-cell response was observed when applying Ad5.SIVGag (see Figure 21B above). When the antigen was changed to SHIVEnv, still no statistically significant effects on the T-cell response towards the antigen could be observed: see Figure 23B. In line with the observation with Ad49.SIVGag (see Figure 21C above that showed a decrease in T cell response when the adjuvant was used), also adding aluminium phosphate to Ad49.SHIVEnv vectors appeared to negatively influence the T-cell response to the SHIVEnv antigen, see Figure 23C, which effect was statistically significant for the two higher doses.

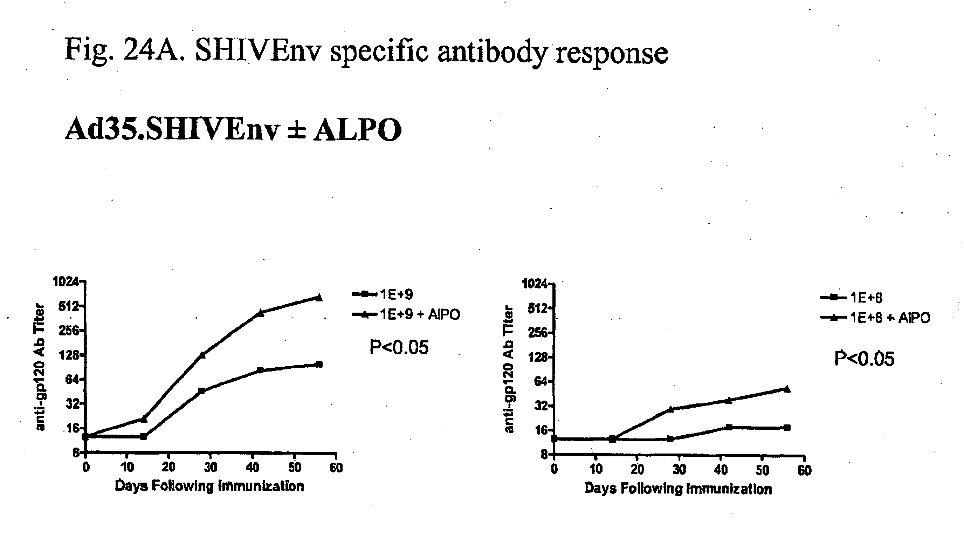

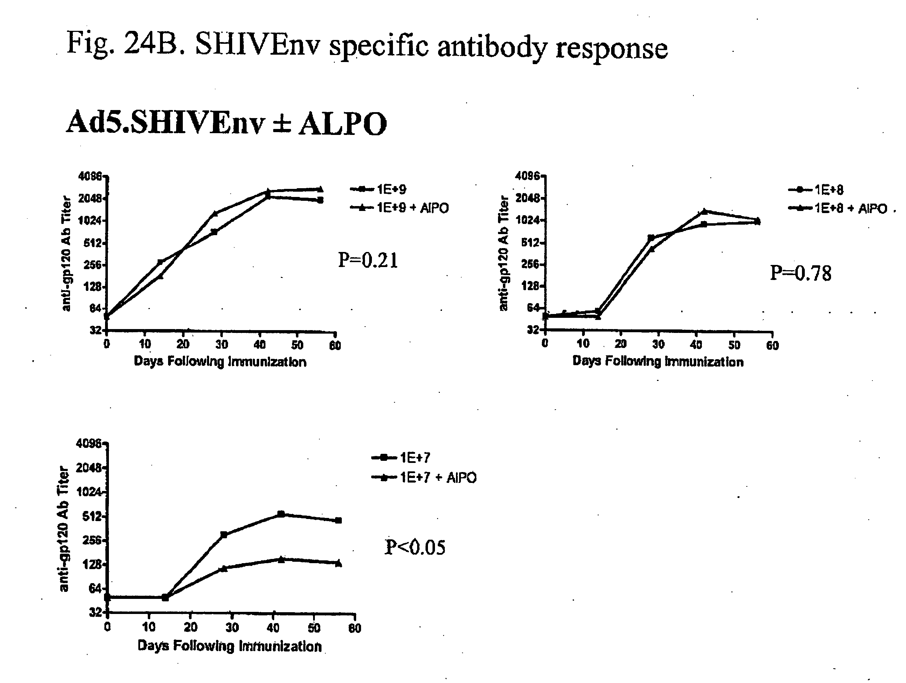

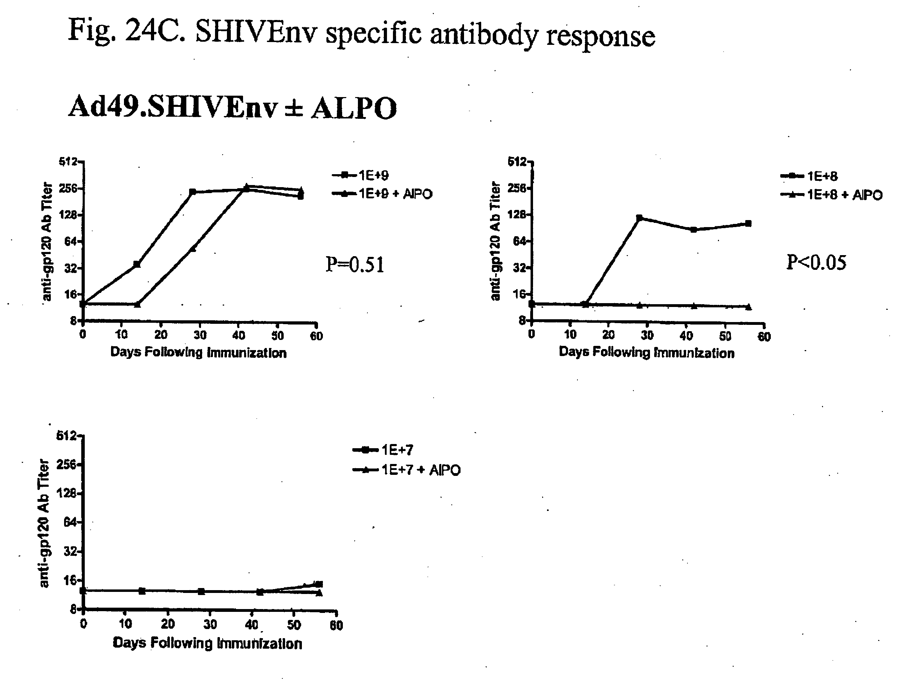

Next, the antibody response towards the SHIVEnv antigen was determined after application of the three vectors Ad35.SHIVEnv, Ad5.SHIVEnv and Ad49.SHIVEnv in different doses. Figure 24A shows that, when Ad35 is used, there is a. significant increase in antibody response against the SHIVEnv antigen when applying $10^8$ and $10^9$ vp. In contrast, when Ad5.SHIVEnv was used, no significant effect is observed with the two lowest doses, although actually a significant decrease in antibody response is found when $10^7$ vp are injected in combination with aluminium phosphate, see Figure 24B. This effect is even more striking when the mice are injected with Ad49.SHIVEnv: at the $10^8$ vp dose, a proper antibody response is found towards the antigen when no adjuvant is present. However, when aluminium phosphate is added to the recombinant vector, the antibody response is completely absent, see Figure 24C. The conclusion from all these comparative experiments between Ad35-, Ad5- and Ad49-based vectors carrying different antigens is that when Ad35-based vectors are used, beneficial properties are found in the presence of aluminium phosphate when each of the antigens (CS, SIVGag, or SHIVEnv is used). In contrast, Ad49-based vectors seem to be hampered in their immunogenic effects, whereas Ad5-based vectors do not benefit in general from adding this adjuvant. '

It was also tested whether the response observed with aluminium phosphate could be further stimulated in a homologous prime/boost setting in which a composition of $10^8$ vp Ad35.CS with 0.5 mg/ml aluminium phosphate could boost either a composition with Ad35.CS alone or a composition with Ad35.CS plus 0.5 mg/ml aluminium phosphate. For this, 5 different Balb/c mouse groups were injected with either one of the following regimens (priming at t = 0, boosting at t = 4 weeks), blood sampling just before boosting and sacrifice at week 8:

| Group | Prime | Boost |
|---|---|---|
| 1 | Ad35.CS | none |
| 2 | Ad35.CS + ALPO | none |
| 3 | Ad35.CS | Ad35.CS |
| 4 | Ad35.CS | Ad35.CS + ALPO |
| 5 | Ad35.CS + ALPO | Ad35.CS + ALPO |

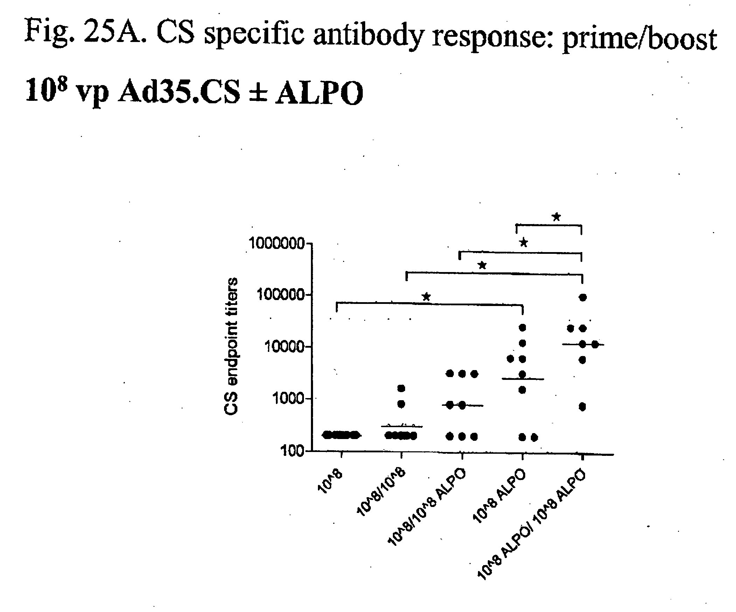

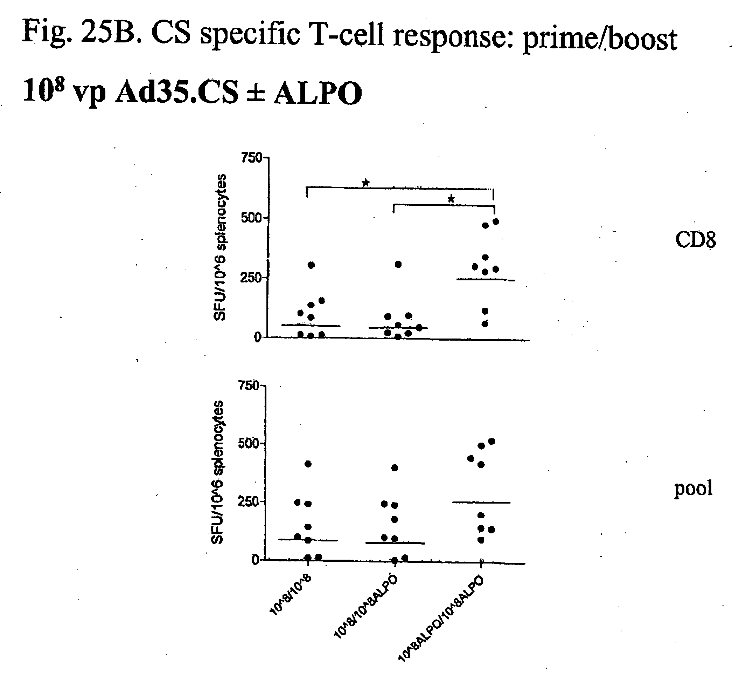

When the antibody response is measured against the CS protein, as described above, it was found that a single immunization with Ad35.CS + ALPO (group 2) provided an increase in response over a prime/boost administration of Ad35.CS without aluminium phosphate (group 3). This effect could be further stimulated in a prime/boost regimen in which both the priming as well as the boosting composition contained aluminium phosphate (group 5), which effect was statistically significant, see Figure 25A. Also the T cell response was significantly boosted in the same experiment when group 5 was compared with group 4, see Figure 25B. It is therefore preferred that when a prime/boost regimen is used, both the priming as well as the boosting composition comprises (next to the recombinant viral vector) the aluminium phosphate adjuvant.

**Example 10. Adsorption of the recombinant adenoviral vector on aluminium hydroxide and aluminium phosphate**

In order to establish whether the recombinant virus actually was adsorbed to the adjuvant compound, a study was performed in which the viral vector Ad35.CS was mixed with the adjuvant in a 1:1 mixture, incubated for 30 min at 4°C and spun down for 5 min at 8000 rpm, upon which the supernatant was taken to determine the number of viral particles not adsorbed to the adjuvant. The viral particles were measured by a Q-PCR method as follows:

Quantitative PCR reagents (TaqMan® 1000RXN Gold with Buffer A Pack) were purchased at Applied Biosystems (Foster City, CA). Sequence detection primers and probe (VIC/TAMRA probe) were designed with Primer Express version 2.0 (Applied Biosystems). Probe was purchased from Applied Biosystems and primers were purchased from Sigma-Genosys (The Woodlands, TX). The primers chosen amplify a product (100 bp) of the' CMV promotor region, which is present as part of the expression cassette in the adenoviruses tested. Forward primer sequence: 5'- CAT CTA CGT ATT AGT CAT CGC TAT TAC CA -3' (SEQ ID NO:5), reverse primer sequence: 5'-TGG AAA TCC CCG TGA GTC A -3' (SEQ ID NO:6) and probe sequence 5'-VIC-ACC GCT ATC CAC GCC CAT TGA TGT -TAMRA-3' (SEQ ID NO:7).

Undigested plasmid DNA (7301 bp) containing the CMV promotor sequence was used as a reference standard for quantitation.

Prior to use the plasmid was subjected to eight 10-fold serial dilutions in $H_2O$, generating a reference standard of $2.0 \times 10^8$ to 20 molecules per PCR reaction. Both plasmid DNA and an adenovirus were used as internal controls. Samples were diluted 100-fold using the appropriate formulation buffer prior to analysis. Subsequently, 5 $\mu$l of 5% deoxycholine (DOC) was added to 45 $\mu$l diluted adenovirus.

Then 45 $\mu$l of DNase mastermix, consisting of 5 $\mu$l 10x DNase buffer (New England Biolabs), 5 $\mu$l DNaseI (Roche) and 35 $\mu$l $H_2O$, was added to 5 $\mu$l DOC treated sample. For the plasmid internal control DNaseI was substituted by $H_2O$ in this step.

Samples were incubated for 30 min at 37°C. To stop the DNaseI reaction, 3 $\mu$l of 20mM EGTA was added to all samples and controls and by a heat inactivation step of 15 min at 95°C, 5 min at 4°C and 15 min at 95°C. As a last step 5 $\mu$l of Proteinase K (Invitrogen) was added and incubated for 60 min at 50°C and followed by a 20 min 95°C incubation. Both the internal virus control and plasmid DNA were not treated with 5% DOC during sample preparation. The PCR mix was prepared as follows: 9.32 $\mu$l $H_2O$, 2 $\mu$l 2.5mM dNTPs, 2.5 $\mu$l 10x buffer A, 3 $\mu$l 25 mM $MgCl_2$, 60nM Forward primer, 60nM Reverse primer, 200nM probe, 0.125 $\mu$l of 5 units/ $\mu$l TaqMan Gold.

The required number of wells of a 96-wells PCR optical plate were filled with 20 $\mu$l of PCR mix. Following sample preparation, 5 $\mu$l of each standard point, sample and internal control was transferred in duplicate to a 96-well PCR optical plate containing the PCR mix. The PCR amplification program was as follows: 95°C for 10 min, 1 cycle; 95°C for 15 sec and 60°C for 1 min; 45 cycles. Data were analyzed with the ABI 7700 Sequence Detection System (SDS) software. The baseline was set from cycle 3 to 10, and the threshold was set at the linear phase of amplification. Raw PCR results (Ct-values) were exported to Microsoft Excell in order to correct the obtained titer for the dilution factor introduced during sample handling.

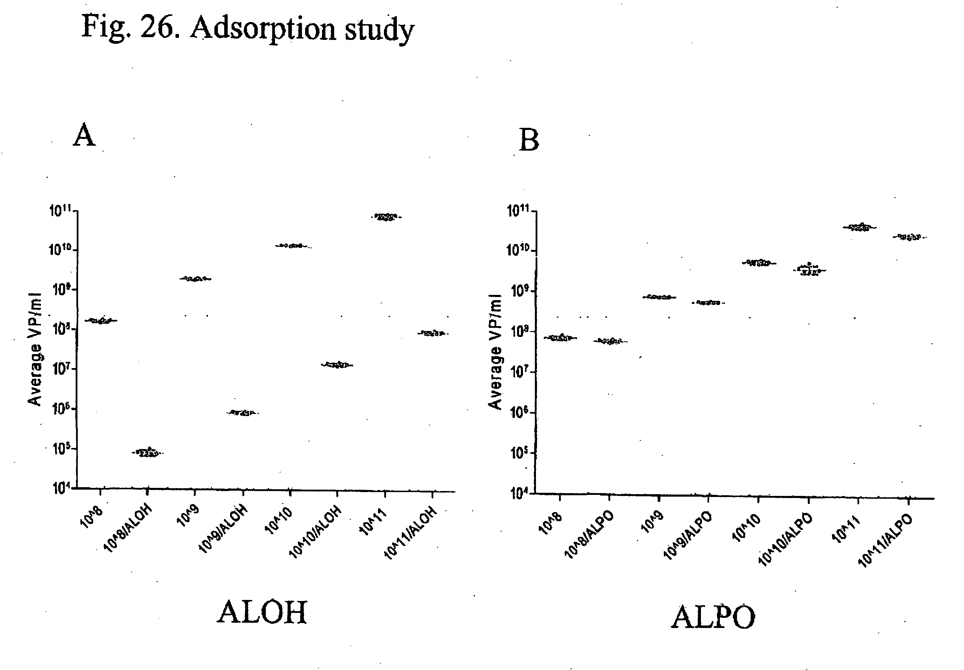

The results are shown in Figure 26. Clearly, the Ad35.CS recombinant vector does not adsorb to aluminium phosphate (B), whereas there is a clear decrease of viral particles in the samples in which the viral particles were mixed with aluminium hydroxide (A). It is concluded that adsorption to the aluminium phosphate adjuvant is not required for the immunopotentiating actions towards the antigen encoded by the Ad35 viral vector.

The data obtained demonstrate that aluminium phosphate does not bind to the Ad35 carrier. Furthermore, aluminium phosphate has no detrimental effect on any of the biological pathways normally exerted by the Ad35 vector and critical for vaccine activity of the vector. The absence of binding is not surprising since prediction studies regarding the iso-electric point of the most abundant Ad35 capsid protein, i.e. hexon (www.expasy.org/links.html) indicated that the Ad35 hexon is negatively charged. Therefore, it was anticipated that in the formulation buffer used (TRIS buffer, pH 8), wherein aluminium phosphate is also negatively charged both components would carry negative charge and thus expel each other. The lack of direct interaction between aluminium phosphate and Ad35 in turn might explain the data demonstrating no negative effect on vector viability. This given the fact that one could only expect detrimental effects on cell attachment, entry, transcription and expression if the adjuvant would in any way be bound to the vector thereby interfering with either receptor recognition, internalization via coated pits, escape from the cellular endosome or genomic DNA transport to the nucleus. Although aluminium-based adjuvants have been used for decades, the precise mechanism of their action is poorly understood. For instance, it has been generally accepted for many years that the antigen should be adsorbed up to at least 70% by aluminium containing adjuvant in order to have a valid vaccine (Aprile MA and Wardlaw (1966). Can J Public Heal th 57:343-60*; World Health Organization. (1976) Technical Report Series No. 595:p. 6-8). However recent data support the hypothesis that the degree of absorption may change upon either intramuscular or subcutaneous injection (Chang et al (2001) Vaccine 19:2884-9*; Iyer et al (2004) Vaccine 22:1475-9*; Jiang et al (2006) vaccine 24:1665-9*: Morefield et al (2005) Vaccine 23:1502-6*; Shi et al (2001) Vaccine 20:80-5*) and that the antigen within the complex of antigen with aluminium adjuvant might be replaced by alternative proteins present in interstitial fluid. Furthermore, studies with DNA immunization have shown the abrogation of vaccine potency when physically bound to aluminium adjuvant, a distinct difference from conventional vaccines in which protein antigens are generally bound to alum.

Although the mechanism thus far is unknown, one can speculate about the way the Ad35 mediated immune response is improved upon aluminium phosphate adjuvation. Given the observation that no complex is formed between carrier and adjuvant, the formation of an antigen-depot at the site of immunization, from where antigen is released slowly does not seem to occur. This leaves a number of alternative possibilities including:

(i) the aluminium phosphate adjuvant increases the inflammatory response leading to the attraction of antigen presenting cells (APC) to the site of injection which increases locally a number of possible target cells for Ad35.CS infection, (ii) the adjuvant increases macropinocytosis thereby increasing the uptake of Ad35.CS by antigen presenting cells, (iii) the adjuvant increases locally the cell surface expression of major histocompatibility complex (MHC) II molecules and co-stimulatory molecules on immune stimulatory cells and thus increases efficiency of the induction of immune response, (vi) the adjuvant binds to the newly synthesized CS protein after it is expressed and secreted from transduced cells and as such acts as classical protein adjuvant, or combinations of two or more of the above. The latter is less probable since it is well established that non-complexed aluminium phosphate is rapidly cleared and that at least a couple of hours is required for the Ad35 vector to infect cells and to allow cells to express transgenic protein. The first hypothesis seems most plausible given the knowledge that aluminium adjuvants are known to recruit inflammatory cells to the site of injection.

**Example 11. Effect of aluminium phosphate in a composition comprising an Ad35 vector harboring two antigens from *P. falciparum***

The effect of the aluminium phosphate adjuvant in immunostimulating an antigen encoded by a nucleic acid carried in the genome of an Ad35-based recombinant virus, was studied. A replication-defective adenovirus, based on Ad35, comprising a genome carrying two antigens from *P. falciparum* was used. The two nucleic acids are linked such that the two encoded proteins, the CS antigen as described above, and the Liver Specific Antigen-1 (LSA-1) protein (Kurtis JD et al (2001) Trends in Parasitology 17:219-223*)* are expressed as a single transcript giving rise to a fusion protein. The amino acid sequence of the full-length encoded fusion protein is as follows:

```
MMRKLAILSVSSFLFVEALFQEYQCYGSSSNTRVLNELNYDNAGTNLYNELEMNYYGKQE
NWYSLKKNSRSLGENDDGNNNNGDNGREGKDEDKRDGNNEDNEKLRKPKHKKLKQPADGN
PDPNANPNVDPNANPNVDPNANPNVDPNANPNANPNANPNANPNANPNANPNANPNANPN
ANPNANPNANPNVDPNANPNANPNANPNANPNANPNANPNANPNANPNANPNANPNANPN
ANPNANPNANPNANPNANPNANKNNQGNGQGHNMPNDPNRNVDENANANSAVKNNNNEEP
SDKHIKEYLNKIQNSLSTEWSPCSVTCGNGIQVRIKPGSANKPKDELDYANDIEKKICKM
EKCSSVFNVVNS
NSEKDEIIKSNLRSGSSNSRNRINEEKHEKKHVLSHNSYEKTKNNENNKFFDKDKELTMS
NVKNVSQTNFKSLLRNLGVSENIFLKENKLNKEGKLIEHIINDDDDKKKYIKGQDENRQE
DLEEKAAQQSDLEQERALAKEKLQEKADTKKNLERKKEHGDVLAEDLYGRLEIPAIELPS
ENERGYYIPHQSSLPQDNRGNSRDSKEISIIEKTNRESITTNVEGRRDIHKGHLEEKKDG
SIKPEQKEDKSADIQNHTLETVNISDVNDFQISKYEDEISAEYDDSLIDEEEDDEDLDEF
KPIVQYDNFQDEENIGIYKELEDLIEKNENLDDLDEGIEKSSEELSEEKIKKGKKYEKTK
DNNFKPNDKSLYDEHIKKYKNDKQVNKEKEKFIKSLFHIFDGDNEILQIVDELSEDITKY
FMKL (SEQ ID NO:8).
```

The CS protein is represented by aa 1-372, whereas the LSA-1 protein is represented by aa 373-796. The first amino acid of LSA-1 (N) given in bold and is underlined. The construction and production of this adenovirus (Ad35.CL) was as outlined for Ad35.CS.

In total 7 groups of 10 mice were injected with the following compositions, in the following amounts:

Ad35.CL $10^8$ + ALPO
Ad35.CL $10^9$ + ALPO
Ad35.CL $10^{10}$ + ALPO
Ad35.CL $10^8$
Ad35.CL $10^9$
Ad35.CL $10^{10}$
Ad35.empty $10^{10}$ (negative control)

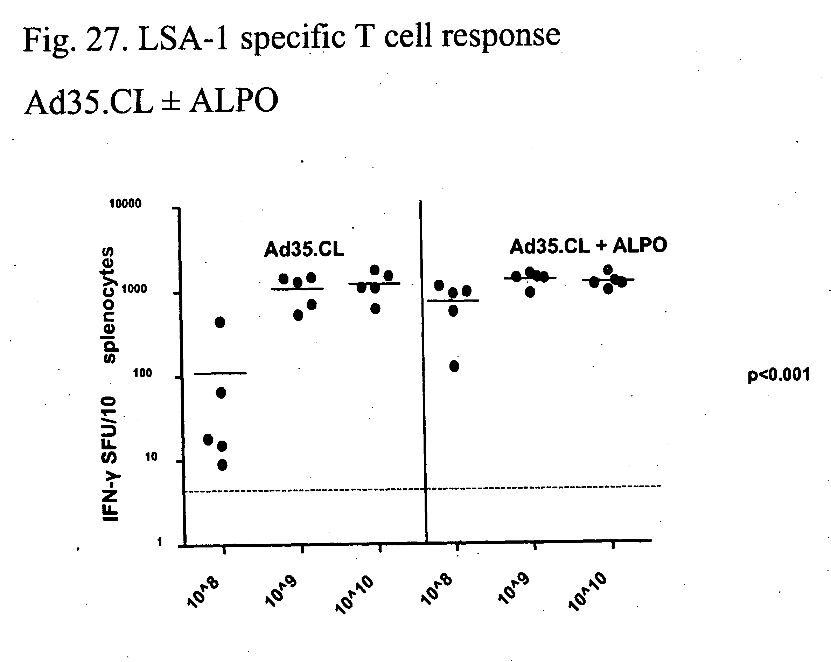

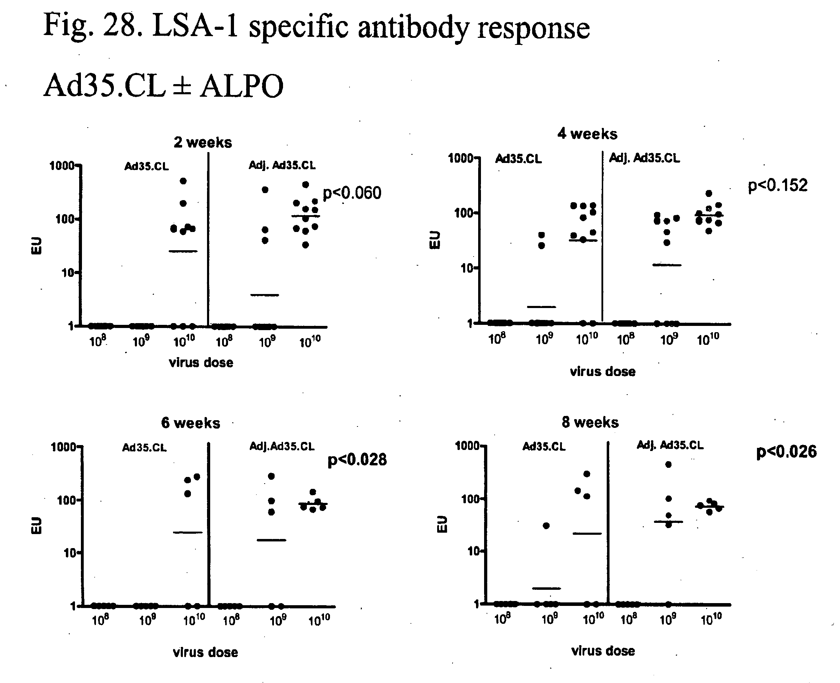

5 mice of each group were sacrificed 4 weeks after injection, while the remaining mice were sacrificed 8 weeks after injection. Blood samples were also taken at week 1.5, 3 and 6. Blood samples were used for antibody responses in an ELISA, while spleens from the sacrificed mice were used in an ELISPOT assay using a CD8 immunodominant peptide identified in CS and in LSA-1, as outlined above and known to the person skilled in the art. The results obtained with respect with CS were in line with the results obtained with the single vector Ad35.CS as described in the examples above (data not shown). Figure 27 shows the LSA-1 specific T cell response after 4 weeks in mice that were injected with Ad35.CL with or without aluminium phosphate. There is a significant increase in the case of the lower dose ($10^7$ vp), when aluminium phosphate is added to the composition. Figure 28 shows the LSA-1 specific antibody response after 2, 4, 6 and 8 weeks. The increase in response is clearly detectable for the separate viral doses used. For instance, at 6 and 8 weeks, the antibody titers for the $10^8$ vp dose are low when no adjuvant is used. However, when the aluminium phosphate was present in the composition used for this dose, a significant increase in antibody titer is observed.

It is concluded that aluminium phosphate has a positive effect on the immune response towards an antigen encoded by a recombinant virus, when that virus is based on human adenovirus serotype 35 (Ad35). The positive response can either be found in antibody titers towards the antigen or T cell responses, or both and do not seem to be antigen-specific, when it is applied in the context of Ad35. However, when another serotype is used, such as Ad5 or Ad49 the effects may be lost (no change in immune response, e.g. in the case of Ad5) or even decreased (as found with some of the immunizations using Ad49), depending on the dose and antigen. It remains to be seen which serotypes can benefit from the addition of aluminium phosphate. However, with the teaching disclosed by the present invention, the skilled person is now provided with the tools to determine which serotype may be useful in one composition with aluminium phosphate and which serotypes should be avoided, when adding an adjuvant is contemplated.

Together, the data as disclosed herein show that the adjuvant effect of aluminium phosphate in combination with a recombinant adenoviral vector, either in a T-cell response or in an antibody response towards the encoded antigen, or in a NAb response towards the viral vector itself, is not generally applicable as it is not found in all different settings. Surprisingly, the effect is specific for the adenovirus serotype used: Ad35-based vectors benefit from the effect, while Ad5-based vectors do not seem to be influenced, and strikingly, Ad49-based vectors are hampered in providing an immune response when applied together with aluminium phosphate.

When the Circumsporozoite antigen (CS) from *P. falciparum* or the SIVGag antigen from Simian Immunodeficiency Virus was used in an Ad35 recombinant backbone, the positive stimulating immunogenic effects on antibody and T cell response was significant. When another antigen, SHIVEnv, was used in the Ad35 vector context, the positive effect was only observed with respect to antibody response.

**Claims**

1.  A composition comprising:

    - a recombinant replication-defective serotype 35 adenovirus (Ad35) comprising an adenoviral genome, said genome comprising a heterologous nucleic acid encoding an immunogenic determinant; and
    - an aluminium-phosphate based adjuvant.

2.  A composition according to claim 1, wherein said immunogenic determinant is the circumsporozoite (CS) protein, or an immunogenic part thereof, from a malaria-causing pathogen.

3.  A composition according to claim 1, wherein said immunogenic determinant is the LSA-1 protein, or an immunogenic part thereof, from a malaria-causing pathogen.

4.  A composition according to claim 2, wherein said immunogenic determinant further comprises the LSA-1 protein, or an immunogenic part thereof, from a malaria-causing pathogen.

5.  A composition according to any one of claims 1-4, wherein said replication-defective adenovirus comprises an adenoviral genome comprising a deletion in the E1 region rendering the adenovirus replication-defective, and further preferably comprising a deletion of the E3 region.

6. A composition according to claim 5, wherein said genome further comprises an E4orf6 region from adenovirus serotype 5.

7. A composition according to any one of claims 1-6, further comprising a pharmaceutically acceptable excipient and/or diluent.

8. A composition according to any one of claims 1-7 for use in the diagnosis, prophylaxis or treatment of malaria.

9. An aluminium phosphate adjuvant and a recombinant replication-defective Ad35, for use in the diagnosis, prophylaxis or treatment of an infectious disease.

10. An adjuvant and Ad35 according to claim 9 for use according to claim 9, wherein said infectious disease is malaria.

11. An adjuvant and Ad35 according to any one of claims 9-10 for use according to claim 9 or 10, wherein said Ad35 comprises an adenoviral genome comprising:

a) a heterologous nucleic acid encoding a CS antigen, or an immunogenic part thereof, of *P. falciparum,* and/or
b) a heterologous nucleic acid encoding an LSA-1 antigen, or an immunogenic part thereof, of *P. falciparum.*

12. An oil-emulsion adjuvant and a recombinant replication-defective adenovirus serotype 35, for use in the diagnosis, prophylaxis or treatment of malaria, wherein said oil-emulsion adjuvant is Covaccine HT or MF59.

13. An adjuvant and adenovirus according to claim 12 for use according to claim 12, wherein said adenovirus comprises an adenoviral genome comprising a heterologous nucleic acid of interest encoding the CS antigen, or an immunogenic part thereof, from *P. falciparum.*

14. A kit of parts for combined use in vaccination, comprising:

- a recombinant replication-defective serotype 35 adenovirus (Ad35) comprising an adenoviral genome, said genome comprising a heterologous nucleic acid encoding an immunogenic determinant; and
- an aluminium-phosphate based adjuvant.

15. A kit of parts according to claim 14, wherein said immunogenic determinant is the CS protein, or an immunogenic part thereof, of *P. falciparum* and optionally
wherein said adenoviral genome further comprises a nucleic acid encoding an LSA-1 protein, or an immunogenic part thereof, of *P. falciparum.*

**Patentansprüche**

1. Zusammensetzung, umfassend:

- ein rekombinantes replikationsdefektes Adenovirus vom Serotyp 35 (Ad35), das ein adenovirales Genom umfasst, wobei das Genom eine heterologe Nucleinsäure umfasst, die eine immunogene Determinante codiert; und
- ein Aluminiumphosphat-basiertes Adjuvans.

2. Zusammensetzung nach Anspruch 1, wobei die immunogene Determinante das Circumsporozoit (CS)-Protein von einem Malaria-verursachenden Pathogen ist, oder ein immunogener Teil davon.

3. Zusammensetzung nach Anspruch 1, wobei die immunogene Determinante das LSA-1-Protein von einem Malaria-verursachenden Pathogen ist, oder ein immunogener Teil davon.

4. Zusammensetzung nach Anspruch 2, wobei die immunogene Determinante zudem das LSA-1-Protein von einem Malaria-verursachenden Pathogen umfasst, oder ein immunogener Teil davon.

**5.** Zusammensetzung nach einem der Ansprüche 1 bis 4, wobei das replikationsdefekte Adenovirus ein adenovirales Genom umfasst, das eine Deletion in der E1-Region umfasst, die das Adenovirus replikationsdefekt macht, und des Weiteren vorzugsweise eine Deletion der E3-Region umfasst.

**6.** Zusammensetzung nach Anspruch 5, wobei das Genom des Weiteren eine E4 or f6-Region vom Adenovirus-Serotyp-5 umfasst.

**7.** Zusammensetzung nach einem der Ansprüche 1 bis 6, das des Weiteren einen pharmazeutisch verträglichen Exzipienten und/oder ein pharmazeutisch verträgliches Verdünnungsmittel umfasst.

**8.** Zusammensetzung nach einem der Ansprüche 1 bis 7 zur Verwendung in der Diagnose, Vorbeugung oder Behandlung von Malaria.

**9.** Aluminiumphosphatadjuvans und ein rekombinantes replikationsdefektes Ad35 zur Verwendung in der Diagnose, Vorbeugung oder Behandlung einer ansteckendenen Erkrankung.

**10.** Adjuvans und Ad35 nach Anspruch 9 zur Verwendung nach Anspruch 9, wobei die ansteckende Erkrankung Malaria ist.

**11.** Adjuvans und Ad35 nach einem der Ansprüche 9 bis 10 zur Verwendung nach Anspruch 9 oder 10, wobei das Ad35 ein adenovirales Genom umfasst, das umfasst:

a) eine heterologe Nucleinsäure, die ein CS-Antigen aus *P. falciparum* oder einen immunogenen Teil davon codiert, und/oder

b) eine heterologe Nucleinsäure, die ein LSA-1-Antigen aus *P. falciparum* oder einen immunogenen Teil davon codiert.

**12.** Öl-Emulsionsadjuvans und ein rekombinantes replikationsdefektes Adenovirus vom Serotyp 35 zur Verwendung in der Diagnose, Vorbeugung oder Behandlung von Malaria, wobei das Öl-Emulsionsadjuvans der Co-Impfstoff HT oder MF59 ist.

**13.** Adjuvans und Adenovirus nach Anspruch 12 zur Verwendung nach Anspruch 12, wobei das Adenovirus ein adenovirales Genom umfasst, das eine heterologe Nucleinsäure von Interesse umfasst, die das CS-Antigen aus *P. falciparum* oder einen immunogenen Teil davon codiert.

**14.** Kit zur kombinierten Verwendung in der Impfung, umfassend:

- ein rekombinantes replikationsdefektes Adenovirus vom Serotyp 35 (Ad35), das ein adenovirales Genom umfasst, wobei das Genom eine heterologe Nucleinsäure umfasst, die eine immunogene Determinante codiert; und
- ein Aluminiumphosphat-basiertes Adjuvans.

**15.** Kit nach Anspruch 14, wobei die immunogene Determinante das CS-Protein aus *P. falciparum,* oder ein immunogener Teil davon ist, und wobei das adenovirale Genom gegebenenfalls des Weiteren eine Nucleinsäure umfasst, die das LSA-1-Protein aus *P. falciparum,* oder einen immunogenen Teil davon codiert.

**Revendications**

**1.** Composition comprenant :

- un adénovirus de sérotype 35 (Ad35) recombiné défectif pour la réplication comprenant un génome adénoviral, ledit génome comprenant un acide nucléique hétérologue codant pour un déterminant immunogène ; et
- un adjuvant à base de phosphate d'aluminium.

**2.** Composition selon la revendication 1, dans laquelle ledit déterminant immunogène est la protéine circumsporozoïte (CS), ou une partie immunogène de celle-ci, provenant d'un agent pathogène responsable du paludisme.

**3.** Composition selon la revendication 1, dans laquelle ledit déterminant immunogène est la protéine LSA-1, ou une partie immunogène de celle-ci, provenant d'un agent pathogène responsable du paludisme.

**4.** Composition selon la revendication 2, dans laquelle ledit déterminant immunogène comprend en outre la protéine LSA-1, ou une partie immunogène de celle-ci, provenant d'un agent pathogène responsable du paludisme.

**5.** Composition selon l'une quelconque des revendications 1 à 4, dans laquelle ledit adénovirus défectif pour la réplication comprend un génome adénoviral comprenant une délétion dans la région E1 rendant l'adénovirus défectif pour la réplication, et comprenant en outre de préférence une délétion de la région E3.

**6.** Composition selon la revendication 5, dans laquelle ledit génome comprend en outre une région E4orf6 d'un adénovirus de sérotype 5.

**7.** Composition selon l'une quelconque des revendications 1 à 6, comprenant en outre un excipient et/ou un diluant pharmaceutiquement acceptable.

**8.** Composition selon l'une quelconque des revendications 1 à 7 pour une utilisation dans le diagnostic, la prophylaxie ou le traitement du paludisme.

**9.** Adjuvant de phosphate d'aluminium et un Ad35 recombiné défectif pour la réplication, pour une utilisation dans le diagnostic, la prophylaxie ou le traitement d'une maladie infectieuse.

**10.** Adjuvant et Ad35 selon la revendication 9, pour une utilisation selon la revendication 9, dans laquelle ladite maladie infectieuse est le paludisme.

**11.** Adjuvant et Ad35 selon l'une quelconque des revendications 9 à 10, pour une utilisation selon la revendication 9 ou 10, dans laquelle ledit Ad35 comprend un génome adénoviral comprenant :

a) un acide nucléique hétérologue codant pour un antigène de CS, ou une partie immunogène de celui-ci, de *P. falciparum,* et/ou
b) un acide nucléique hétérologue codant pour un antigène de LSA-1, ou une partie immunogène de celui-ci, de *P. falciparum.*

**12.** Adjuvant pour émulsion de type huileux et un adénovirus de sérotype 35 recombiné défectif pour la réplication, pour une utilisation dans le diagnostic, la prophylaxie ou le traitement du paludisme, dans laquelle ledit adjuvant pour émulsion de type huileux est la Covaccine HT ou MF59.

**13.** Adjuvant et adénovirus selon la revendication 12, pour une utilisation selon la revendication 12, dans laquelle ledit adénovirus comprend un génome adénoviral comprenant un acide nucléique hétérologue d'intérêt codant pour l'antigène CS, ou une partie immunogène de celui-ci, provenant de *P. falciparum.*

**14.** Kit de pièces pour une utilisation combinée dans une vaccination comprenant :

- un adénovirus de sérotype 35 (Ad35) recombiné défectif pour la réplication comprenant un génome adénoviral, ledit génome comprenant un acide nucléique hétérologue codant pour un déterminant immunogène ; et
- un adjuvant à base de phosphate d'aluminium.

**15.** Kit de pièces selon la revendication 14, dans lequel ledit déterminant immunogène est la protéine CS, ou une partie immunogène de celle-ci, de *P. falciparum* et éventuellement dans lequel ledit génome adénoviral comprend en outre un acide nucléique codant pour une protéine LSA-1, ou une partie immunogène de celle-ci, de *P. falciparum.*

Fig 1. Aluminium hydroxide (ALOH)

# Fig. 2. Aluminium phosphate (ALPO)

## A.

□ Cell viability

△ eGFP expression

[ALPO mg/ml]

## B.

■ VP/cell

[ALPO mg/ml]

Fig. 3. Covaccine™ HT

EP 1 998 804 B1

Fig. 4. MF-59

# Fig. 5A. CS specific T cell response - ALOH

# Fig. 5B. CS specific T cells response - ALOH

## - ALOH

EP 1 998 804 B1

**4 wks**

SFU/10e6 splenocytes

1400
1200
1000
800
600
400
200
0

10e7  10e8  10e9  10e10  Empty

## + ALOH

SFU/10e6 splenoytes

1400
1200
1000
800
600
400
200
0

10e7  10e8  10e9  10e10  Empty

**8 wks**

SFU/10e6 splenocytes

1400
1200
1000
800
600
400
200
0

10e7  10e8  10e9  10e10  Empty

SFU/10e6 splenocytes

1400
1200
1000
800
600
400
200
0

10e7  10e8  10e9  10e10  Empty

Readout peptide pool

Fig. 6A. CS specific T cell response - ALPO

# Fig. 6B. CS specific T cell response - ALPO

## Fig. 7. CS specific antibody response - ALOH

- ALOH

+ ALOH

4 wks

8 wks

Fig. 8. CS antibody response - ALPO

# Fig. 9. Vector T cell response - ALOH

# Fig. 10. Vector T cell response - ALPO

Fig. 11. Vector specific antibody response - ALOH

4 wks

8 wks

# Fig. 12. Vector specific antibody responses - ALPO

4 wks

8 wks

# Fig. 13A. CS specific T cell response - Covaccine™ HT

**- HT**

**+ HT**

4 wks

8 wks

Readout dominant epitope

EP 1 998 804 B1

# Fig. 13B. CS specific T cell response - Covaccine™ HT

- HT

+ HT

4 wks

8 wks

Peptide pool

# Fig. 14A. CS specific T cell responses - MF-59

## - MF-59

## + MF-59

Readout dominant epitope

# Fig. 14B. CS specific T cell response - MF-59

- MF-59

+ MF-59

4 wks

8 wks

Peptide pool

EP 1 998 804 B1

# Fig. 15. CS specific antibody response - Covaccine™ HT

- HT                                          + HT

4 wks

8 wks

EP 1 998 804 B1

Fig.16. CS specific antibody response - MF-59

# Fig. 17. Vector T cell response - Covaccine™ HT

EP 1 998 804 B1

Fig. 18. Vector T cell response - MF-59

# Fig.19. Vector specific antibody response - Covaccine™ HT

# Fig.20. Vector specific antibody response - MF-59

EP 1 998 804 B1

## Fig. 21A. SIVGag specific T-cell response - ALPO Ad35

## Fig. 21B. SIVGag specific T-cell response - ALPO Ad5

EP 1 998 804 B1

Fig. 21C. SIVGag specific T-cell response - ALPO
Ad49

# Fig. 22A. Neutralizing antibody titers

## Ad35.SIVGag ± ALPO

EP 1 998 804 B1

# Fig. 22B. Neutralizing antibody titers

## Ad5.SIVGag ± ALPO

# Fig. 22C. Neutralizing antibody titers

## Ad49.SIVGag ± ALPO

EP 1 998 804 B1

# Fig. 23A. SHIVEnv specific T-cell response

## Ad35.SHIVEnv ± ALPO

Fig. 23B. SHIVEnv specific T-cell response

Ad5.SHIVEnv ± ALPO

# Fig. 23C. SHIVEnv specific T-cell response

## Ad49.SHIVEnv ± ALPO

# Fig. 24A. SHIVEnv specific antibody response

## Ad35.SHIVEnv ± ALPO

EP 1 998 804 B1

# Fig. 24B. SHIVEnv specific antibody response

## Ad5.SHIVEnv ± ALPO

P=0.21

P=0.78

P<0.05

EP 1 998 804 B1

# Fig. 24C. SHIVEnv specific antibody response

## Ad49.SHIVEnv ± ALPO

P=0.51

P<0.05

EP 1 998 804 B1

Fig. 25A. CS specific antibody response: prime/boost
$10^8$ vp Ad35.CS ± ALPO

# Fig. 25B. CS specific T-cell response: prime/boost
## $10^8$ vp Ad35.CS ± ALPO

# Fig. 26. Adsorption study

### A

ALOH

### B

ALPO

EP 1 998 804 B1

Fig. 27. LSA-1 specific T cell response

Ad35.CL ± ALPO

# Fig. 28. LSA-1 specific antibody response

## Ad35.CL ± ALPO

EP 1 998 804 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2005055984 W
- WO 2004055187 A
- WO 9014837 A
- WO 03037275 A
- EP 0833923 B1
- WO 2006053871 A
- WO 03104467 A
- WO 0070071 A
- WO 9700326 A
- WO 9955132 A
- WO 9964582 A
- WO 0003029 A
- WO 04055187 A
- WO 0240665 A

### Non-patent literature cited in the description

- **SHIVER et al.** *Nature,* 2002, vol. 415, 331-335
- **SULLIVAN et al.** *Nature,* 2000, vol. 408, 605-609
- **HEPPNER et al.** *Vaccine,* 2005, vol. 23, 2243-2250
- **OPHORST et al.** *Infect. Immun.,* 2006, vol. 74, 313-320
- Vaccine Design. Plenum, 1995
- **PODDA A.** *Vaccine,* 2001, vol. 19, 2673-2 680
- **BLOM A.G. ; HILGERS L.A.TH.** *Vaccine,* 2004, vol. 23, 743-754
- **HIGGINS D.A. et al.** *Vaccine,* 1996, vol. 14, 478-484
- **VERHAAGH S. et al.** *J Gen Virol,* 2006, vol. 87, 255-265
- **KLEIN et al.** *Gene Ther,* 2000, vol. 7, 458-463
- **OTT et al.** *Vaccine,* 1995, vol. 13, 1557-1562
- **BAROUCH DH et al.** *J. Immunol,* 2004, vol. 172, 6290-6297
- **OPHORST OJ et al.** *Vaccine,* 2007, vol. 25, 1426-36
- **SPRANGERS MC et al.** *J Clin Microbiol,* 2003, vol. 41, 5046-52
- **SPRANGERS MC et al.** *J Clin Microbiol,* 2003, vol. 41, 5046-5052
- **BAROUCH DH et al.** *J Immunol,* 2004, vol. 172, 6290-7
- **LEMCKERT AAC et al.** *J Gen Virol,* 2006, vol. 87, 2891-9
- **REIMANN KA et al.** *J Virol,* 1996, vol. 70, 6922-8
- **APRILE MA ; WARDLAW.** *Can J Public Heal th,* 1966, vol. 57, 343-60
- World Health Organization. *Technical Report Series No. 595,* 1976, 6-8
- **CHANG et al.** *Vaccine,* 2001, vol. 19, 2884-9
- **IYER et al.** *Vaccine,* 2004, vol. 22, 1475-9
- **JIANG et al.** *vaccine,* 2006, vol. 24, 1665-9
- **MOREFIELD et al.** *Vaccine,* 2005, vol. 23, 1502-6
- **SHI et al.** *Vaccine,* 2001, vol. 20, 80-5
- **KURTIS JD et al.** *Trends in Parasitology,* 2001, vol. 17, 219-223